# EUROPEAN PATENT APPLICATION

(11) **EP 3 960 165 A1**
(43) Date of publication of application: **02.03.2022**
(21) Application number: 20192749.8
(22) Date of filing: 25.08.2020
(51) Int. Cl.: A61K 31/166, A61K 31/402, A61K 31/4965

(54) **NHE-1 INHIBITORS FOR THE TREATMENT OF CORONAVIRUS INFECTIONS**

(71) Applicant: Fondation EspeRare, 1202 Geneva (CH); Ares Trading S.A., 1170 Aubonne (CH)
(72) Inventor: PORTE THOME, Florence, 1202 Geneva (CH); KANT MAREDA, Caroline, 1202 Geneva (CH); DURAND AVALLONE, Caroline, 1202 Geneva (CH); MAREDA, Jiri, 1202 Geneva (CH)
(74) Representative: Merck Serono S.A. Intellectual Property

(57) **Abstract**

The present invention encompasses NHE-1 inhibitors for use in the treatment of coronavirus infections, including COVID-19, alone or in combination with one or more additional therapeutic agents.

## Description

### TECHNICAL FIELD OF THE INVENTION

The present invention provides for NHE-1 inhibitors and their use in the treatment of coronavirus infections (including SARS-CoV infections such as COVID-19) and their acute and chronic consequences including life threatening medical complications.

### BACKGROUND OF THE INVENTION

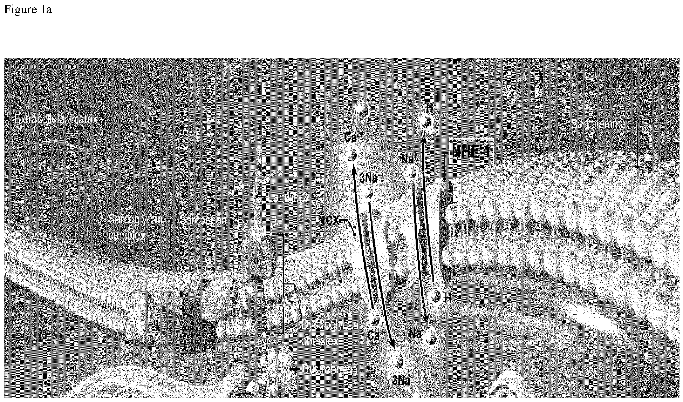

Na+/H+ exchanger type 1 (NHE-1) is the sodium/proton exchanger 1, also named sodium-proton antiporter 1 or SLC9A1 (SoLute Carrier family 9A1), that in humans is encoded by the SLC9A1 gene (Fliegel et al., Molecular and Cellular Biochemistry. 125 (2): 137-43). The sodium-proton antiporter (SLC9A1) is a ubiquitous membrane-bound transporter involved in volume- and pH-regulation of vertebrate cells. It is activated by a variety of signals including growth factors, mitogens, neurotransmitters, tumor promoters and others (Cardone et al., Journal of Molecular Sciences. 20 (15): 3694). In normal conditions, NHE-1 maintains intracellular pH (pHi) and volume by removing one intracellular proton (H+) ion in exchange for a single extracellular sodium (Na+) ion **(Fliegel L.** doi:10.1016/j.biocel.2004.02.006.). See **Figure 1a****.**

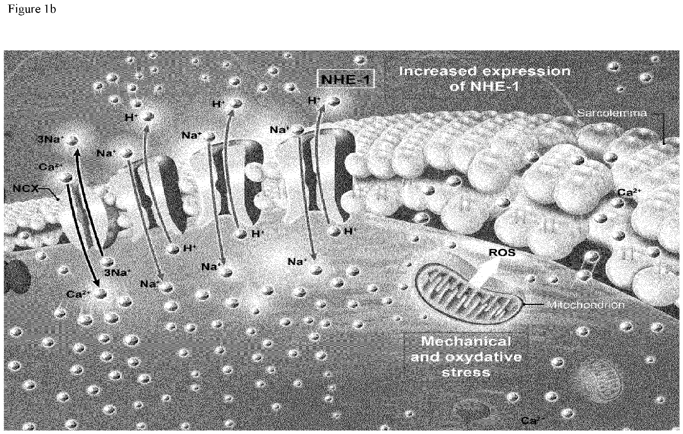

In certain pathological conditions (e.g. heart failure, cardiovascular diseases, diabetes, DMD), NHE-1 is activated, leading to a rapid accumulation of sodium in cells **(Fliegel L.** doi:10.3390/ijms20102378) and an acidification of the extracellular space. The high sodium concentration drives an increase in calcium (Ca2+) via direct interaction and reversal of the Na+/Ca2+ exchanger (NCX). The resulting accumulation of calcium triggers various pathways leading to cell death. **See** **Figure 1b****.** NHE-1 is known to contribute to cardiac hypertrophy **(Odunewu-Aderibigbe** doi: 10.1002/iub.1323).

The concept of NHE-1 involvement in cardiac pathology has been adopted for decades and is supported by a plethora of experimental studies demonstrating effective NHE-1 inhibition in protecting the myocardium against ischemic and reperfusion injury as well as attenuating myocardial remodeling and heart failure **(Evans,** doi:10.1016/j.pmrj.2009.04.010). The cardioprotective effects of NHE-1 inhibitors, including Rimeporide, have been extensively studied in various animal models of myocardial infarction and dystrophic cardiomyopathy including DMD **(Ghaleh,** doi: 10.1016/j.ijcard.2020.03.031). Other preclinical experiments **(Chahine,** doi: 10.1016/j.yjmcc.2005.01.003, **Bkaily,** doi.org/10.1139/cjpp-2017-0265) have underlined the significance of myocardial necrosis, due to pH abnormalities as well as calcium and sodium imbalances in the pathophysiology of heart failure and have demonstrated the beneficial effects of NHE-1 inhibition using Rimeporide in preventing the deleterious effects of Ca2+ and Na+ overload **(Bkaily,** doi.org/10.1139/cjpp-2017-0265).

NHE-1 activation has also been implicated in various diseases, such as myocardial fibrosis, a key pathology in Duchenne Muscular Dystrophy patients leading to dilated cardiomyopathy, the leading cause of death in these patients. Rimeporide, working through NHE-1 inhibition is cardioprotective in both hamsters **(Bkaily,** doi.org/10.1139/cjpp-2017-0265) and golden retriever muscular dystropic dogs **(Ghaleh,** doi: 10.1016/j.ijcard.2020.03.031) with reduction in cardiac pathology including fibrosis, left ventricular function and improved survival in hamsters.

NHE-1 is constitutively active in a neoplastic microenvironment, dysregulating pH homeostasis and altering the survival, differentiation, and proliferation of cancer cells, thereby causing them to become tumorigenic. NHE-1 has been shown to contribute to the growth and metastasis of transformed cells. Karki et al **(**Karki, doi: 10.1074/jbc.M110.165134J Bio Chem, 2011) have established that B-Raf associates with and stimulates NHE-1 activity and that B-RafV600E also increases NHE-1 activity that raises intracellular pH suggesting Rimeporide could be active in melanoma treatment. Other authors have suggested that NHE-1 inhibitors such as Rimeporide could be truly effective anticancer agents in a wide array of malignant tumours including breast cancer, colorectal cancer, NSCLC (non-small lung carcinoma), glioblastoma and leukemia **(**Harguindey et al, Journal of Translational Medicine 2013, 11:282).

In renal diseases, NHE-1 inhibitor abolished Angiotensin II-induced podocyte apoptosis (Liu Y.et al, J of Pharmacol Sci. 2013; 122(3): 176-183), suggesting that Rimeporide could also be beneficial to treat nephrotic syndromes such as focal segmental glomerulosclerosis, diabetic nephropathy (Li **et al**., DOI: 10.1155/2016/1802036) and in general in the progression of renal impairment.

Liu Y et al, (Journal of Neurosci. 2013. 33(31):12656 -12669) have established that misregulation of local axonal ion homeostasis including pH is an important mechanism for axon degeneration and that selective disruption of NHE1-mediated proton homeostasis in axons can lead to degeneration, suggesting that local regulation of pH is pivotal for axon survival.

Hypoxia-induced pulmonary artery hypertension is characterized by elevated pulmonary artery pressure, increased pulmonary vascular resistance, and pulmonary vascular remodeling (Meyrick B. Clin Chest Med. 1983; 4 (2): 199-217). With chronic hypoxia there is a rise in pulmonary artery pressure, pulmonary vascular resistance and a proliferation of pulmonary artery smooth muscle cells. Increased Na+/H+ exchange with an intracellular alkalization is an early event in cell proliferation. This intracellular alkalization by stimulation of Na+/H+ exchange appears to play a permissive role in the pulmonary artery smooth muscle cell (PASMC) proliferation of vascular remodeling. Inhibition of NHE-1 prevents the development of hypoxia-induced vascular remodeling and pulmonary hypertension (Huestch J. 5 (2):228-243; Pulm Circ 2015).

NHE-1 inhibition leads to: Normalization of intracellular sodium, calcium and pH, thus improving cellular function and reducing muscular edema; prevention of progressive congestive heart failure; regulation of inflammatory processes; prevention of fibrosis. NHE-1 inhibitors thus have the potential to address key pathophysiological processes and improve cellular health in DMD (Duchene muscular dystrophy) and heart failure models by restoring ion homeostasis. Known NHE-1 inhibitors are for example rimeporide, cariporide, eniporide which all belong to the class of benzoyl-guanidine derivatives (based on a phenyl ring), or amiloride, EIPA (5-(N-ethyl-N-isopropyl)amiloride), DMA (5-(N,N-dimethyl)amiloride), MIBA (5-(N-methyl-N-isobutyl)amiloride) and HMA 5 -N, N-(hexamethylene)amiloride which belong to the class of pyrazinoyl-guanidine derivatives (based on a pyrazine ring).

The chemical names and chemical structures of some selected NHE-1 inhibitors are as follows.

### Rimeporide: N-(4,5-bismethanesulfonyl-2-methylbenzoyl)guanidine

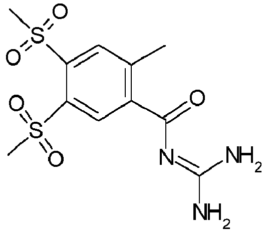

### Cariporide: N-(Diaminomethylidene)-3-methanesulfonyl-4-(propan-2-yl) benzamide

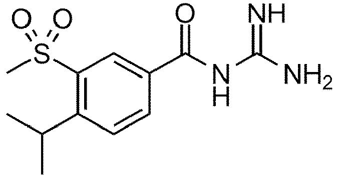

### Eniporide: N-(diaminomethylidene)-2-methyl-5-methylsulfonyl-4-pyrrol-1-ylbenzamide

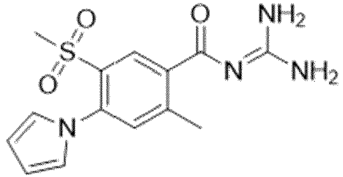

### Amiloride: 3,5-Diamino-N-carbamimidoyl-6-chlorpyrazin-2-carbamid

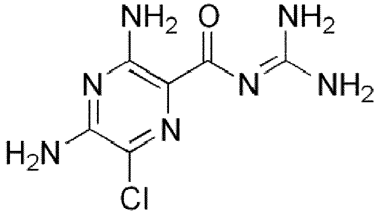

### Coronaviruses

Coronaviruses (CoVs) are positive-sense, single-stranded RNA (ssRNA) viruses of the order *Nidovirales,* in the family *Coronaviridae.* There are four sub-types of coronaviruses - alpha, beta, gamma and delta - with the *Alphacoronaviruses* and *Betacoronaviruses* infecting mostly mammals, including humans. Over the last two decades, three significant novel coronaviruses have emerged which transitioned from a non-human mammal host to infect humans: the severe acute respiratory syndrome (SARS-CoV1) which appeared in 2002, Middle East respiratory syndrome (MERS-CoV) which appeared in 2012, and COVID-19 (SARS-CoV2), a betacoronavirus, which appeared in late 2019. In the first five months of identification of SARS-CoV2, over 17 million people are known to have been infected, and almost 680,000 people are reported to have died. Both numbers likely represent a significant undercount of the devastation brought by the disesase.

Coronaviruses (CoVs) are a group of large and enveloped viruses with positive-sense, single-stranded RNA genomes and they contain a set of four proteins that encapsidate the viral genomic RNA: the nucleocapsid protein (N), the membrane glycoprotein (M), the envelope protein (E), and the spike glycoprotein (S). To enter host cells, coronaviruses first bind to a cell surface receptor for viral attachment. SARS coronavirus (SARS-CoV) uses angiotensin-converting enzyme 2 (ACE2) as a receptor to enter target cells. Subsequently, SARS-CoVs enter the host cells via two routes: (i) the endocytic pathway and (ii) non-endosomal pathway. Among them, the endocytic pathway is of particular importance. (**Shang et al.** doi: 10.1073/pnas.2003138117).

The transmembrane spike (S) glycoprotein at the SARS-CoV surface binds to ACE2 to enter into host cells. S comprises two functional subunits responsible for binding to the host cell receptor (S1 subunit) and fusion of the viral and cellular membranes (S2 subunit). For all CoVs, S is further cleaved by host proteases at the so-called S2' site located immediately upstream of the fusion peptide. This cleavage has been proposed to activate the protein for membrane fusion via extensive irreversible conformational changes. Low pH is required to activate the protease (among which cathepsin L) and ensure endosomal entry. As a conclusion, coronavirus entry into susceptible cells is a complex process that requires the concerted action of receptor-binding and pH-dependent proteolytic processing of the S protein to promote virus-cell fusion. (**Walls** et **al.,** doi.org/10.1016/j.cell.2020.02.058) (White 2016, Fusion of Enveloped Viruses in Endosomes, Traffic 2016; 17: 593-614).

### COVID-19

SARS-CoV-2 closely resembles SARS-CoV-1, the causative agent of SARS epidemic of 2002-03 (Fung, et al, Annu. Rev. Microbiol. 2019. 73:529-57). Severe disease has been reported in approximately 15% of patients infected with SARS-CoV-2, of which one third progress to critical disease (e.g. respiratory failure, shock, or multiorgan dysfunction (**Siddiqi, et al.** doi.org/10.1016/j.healun.2020.03.012, **Zhou, et al.** doi.org/10.1016/S0140-6736(20)30566-3). Fully understanding the mechanism of viral pathogenesis and immune responses triggered by SARS-CoV-2 would be extremely important in rational design of therapeutic interventions beyond antiviral treatments and supportive care.

Severe acute respiratory syndrome (SARS)-Corona Virus-2 (CoV-2), the etiologic agent for coronavirus disease 2019 (COVID-19), and one of the largest viral RNA genomes known (#30kb), has caused a pandemic affecting over seventeen million people worldwide with a case fatality rate of 2-4% as of July 2020. The virus has a high transmission rate, likely linked to high early viral loads and lack of pre-existing immunity (**He**, **et. al**. doi.org/10.1038/s41591-020-0869-5). It causes severe disease especially in the elderly and in individuals with comorbidities such as increased age, cardiac diseases, diabetes and patients with a vulnerable heart. The global burden of COVID-19 is immense, and therapeutic approaches are increasingly necessary to tackle the disease. Intuitive anti-viral approaches including those developed for enveloped RNA viruses like HIV-1 (lopinavir plus ritonavir) and Ebola virus (remdesivir) have been implemented in testing investigational drugs (**Grein et al**. doi.org/10.1056/NEJMoa2007016; **Cao,et al**. doi: 10.1056/NEJMoa2001282). But given that many patients with severe disease present with immunopathology, host-directed immunomodulatory approaches are also being considered, either in a staged approach or concomitantly with antivirals (**Metha, et al.** doi.org/10.1016/S0140-6736(20)30628-0, **Stebbing, et al.** doi.org/10.1016/S1473-3099(20)30132-8).

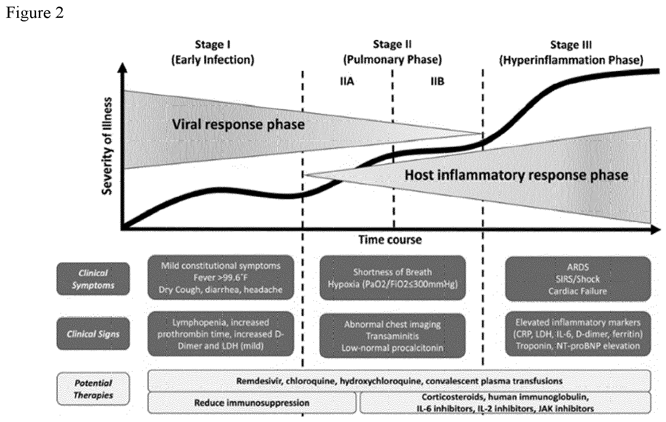

COVID-19 is a spectrum disease, spanning from barely symptomatic infection to critical, life threatening illness. All three coronaviruses (SARS CoV1, MERS CoV and SARS CoV2) induce exhuberant host immune responses that can trigger severe lung pathology, inflammatory cytokine storm, myocardial injury leading to a worse prognosis and ultimately to death in about 10% of patients. **See** **Figure 2****.**

The occurrence and severity of Acute lung Injury (ALI) are a major determining factor of the prognosis of patients with COVID-19 infection. About 30% patients with COVID-19 infection in Intensive Care Unit (ICU) developed severe lung edema, dyspnea, hypoxemia, or even Acute Respiratory Distress Syndrone (ARDS). ARDS is defined clinically by the acute onset of hypoxemia associated with bilateral pulmonary infiltrates (opacities on chest imaging and respiratory), which are not explained by cardiac failure and which can lead to mild, moderate, or severe hypoxemia. The syndrome is characterized by disruption of endothelial barrier integrity and diffuse lung damage. Imbalance between coagulation and inflammation is a predominant feature of ARDS, leading to extreme inflammatory response and diffuse fibrin deposition in vascular capillary bed and alveoli. Activated platelets participate in the complex process of immunothrombosis, which is a key event in ARDS pathophysiology.

Thrombosis has been shown to contribute to increased mortality in COVID-19 patients. It can lead to a pulmonary embolism (PE), which can be fatal, but also higher rates of strokes and heart attacks are observed in patients with thrombosis. This was confirmed in several retrospective studies and provides a rationale for using anticoagulant therapies to prevent from thrombosis.

A recent Dutch study of 184 patients with COVID-19 pneumonia admitted to an intensive care unit (ICU) found a 49% cumulative incidence of thrombotic complications and despite thromboprophylaxis. (**Klok et al.** doi.org/10.1016/j.thromres.2020.04.041). Postmortem studies are finding clots in the capillaries of the lungs in COVID-19 patients, restricting the oxygenated blood from moving through the lungs.

Tang et al. reported on significantly higher D-dimer and poor prognosis in 183 consecutive patients with COVID-19 pneumonia (**Tang et al:** doi: 10.1111/jth.14768]. Those who did not survive their illness compared with survivors had higher D-dimer level as well as other fibrin(ogen) degradation products (FDP). Abnormal coagulation parameters were evident early after hospitalization and in some patients, fibrinogen concentrations and antithrombin activity decreased over time. The same investigators (**Tang et al.** doi: 10.1111/jth.14817) reported in 445 patients that anticoagulant therapy, primarily with low molecular weight heparin (LMWH) administered for 7 days or longerwas associated with a lower 28-day mortality.

**Helms et al.** (doi: 10.1007/s00134-020-06062] reported the occurrence of thrombotic events among 150 patients with COVID-19 and ARDS admitted to the ICU. 16.7% of patients experienced a pulmonary embolism.

**Ackermann et al** (doi: 10.1056/NEJMoa2015432) have observed the lungs from deceased patients with Covid-19 and observed that patients had widespread vascular thrombosis with microangiopathy and occlusion of alveolar capillaries

D-dimer belongs to the fibrin(ogen) degradation products that are involved in platelet activation. Elevated D-Dimer in patients with COVID-19 at the time of hospital admission, is a predictor of the risk of development of ARDS, PE and death. Zhou et al (Lancet. 2020;395(10229):1054-1062) reported that D-dimer levels > 1ug/mL at hospital admission is a predictor of a worse prognosis and of death.

International consensus on the treatment of coagulopathy in patients with COVID-19 calls for low molecular weight heparin (LMWH) or other anticoagulants administered at prophylaxis doses pending the emergence of additional data in patients who are eligible to receive thormboprophylaxis. However, thromboprophylaxis using LMWH/ antiplatelets/ anticoagulants can only be used in patients where the risk of bleeding does not exceed the risk of thrombosis. While many unanswered questions remain about the mechansims of COVID-19-associated coagulopathy, mean platelet volume (as measured by increased platelet volume and size) is another biomarker used in other diseases of platelet function and activation. Some studies also suggest that platelet volume correlates with increased risk for cardiovascular morbidity and mortality. Increased mean platelet volume has been identified as a risk factor in patients with metabolic syndrome, myocardial infarction, ischemic stroke (Tavil et al. Platelets. 2010;21 (5):368-372; Greisenegger et al. Stroke. 2004;35(7):1688-1691). Rimeporide could be a safe approach alone or in combination with LMWH to efficiently thrombotic events in COVID-19 patients.

While still speculative, long-term pulmonary consequences of COVID-19 pneumonia in patients who have recovered may include development of progressive, fibrotic irreversible interstitial lung disease such as interstitial pulmonary fibrosis, or pulmonary hypertension. Small degree of residual but non-progressive fibrosis can result in considerable morbidity and mortality in an older population of patients who had COVID-19, many of whom will have pre-existing pulmonary conditions.

The majority of COVID-19 cases (about 80%) is asymptomatic or exhibits mild to moderate symptoms (fever, fatigue, cough, sore throat and dyspnea..), but approximately 15% progresses to severe pneumonia (**Cantazaro et al.** doi.org/10.1038/s41392-020-0191-1). Excessive inflammatory innate response and dysregulated adaptive host immune defense may cause harmful tissue damage at both at the site of virus entry and at systemic level. Such excessive pro-inflammatory host response in patients with COVID-19 has been hypothesized to induce an immune pathology resulting in the rapid course of acute lung injury (ALI) and ARDS, Cardiogenic Shock or multiorgan failure in particular in patients with high virus load. Prior experience from treating SARS-CoV and MERS-CoV have shown that controlling inflammatory responses through immunomodulators are effective measures to improve the prognosis of HCoV infection (**Arabi et al.** doi: 10.1093/cid/ciz544). This can be achieved by using immunomodulators such as corticosteroids and cytokine antagonists (for example anti IL6) but such treatments carry their own risks to delay the clearance of the virus by inhibiting the ability of the body's immune defense mechanism and ultimately leading to adverse consequences. Increased vascular permeability is also a hallmark change that occurs in the process of a cytokine storm. Drugs aiming at improving vascular permeability or inhibiting the mononuclear/macrophage recruitment and function could also alleviate the storm of inflammatory factors triggered by COVID-19 infection. Such drugs are seen as interesting complement and as a safer therapeutic approach that would not compromise the host immune response and potential delay of virus clearance.

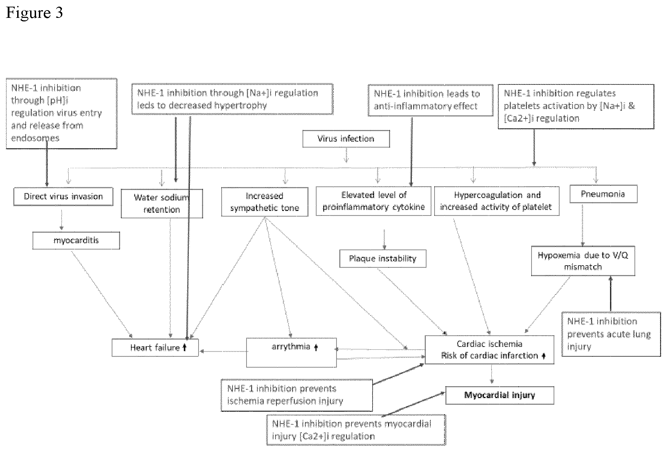

The cardiovascular manifestations induced by COVID-19 infection have generated considerable concern. The overall case fatality rate was 2.3 to 4% but the mortality reached 10.5%in patients with underlying cardiovascular diseases (**Babapoor Farokhran et al.** doi: 10.1016/j.lfs.2020.117723). COVID-19 related heart injury can occur in several ways and can be caused by the virus itself or are a byproduct of the body's reaction to it. See **Figure 3**

First, patients with preexisting cardiac diseases are at greater risk for severe cardiovascular and respiratory complications. Second, people with undiagnosed heart disease may be presenting with previously silent cardiac symptoms unmasked by the viral infection. Third, some patients may experience heart damage mimicking heart attack injury even without preexisting atherosclerosis (myocardial infarction type 2). This scenario occurs when the heart muscle is starved for oxygen which in COVID-19 patients is caused by a mismatch between oxygen spply and oxygen demand due to the pneumonia. The consequences of the lack of oxygen supply result in ischemia of the myocardium leading to ischemia reperfusion injury to the myocardium: Ischemia causes regional scaring of the heart, which is irreversible and can lead to arytthmias. Myocarditis in COVID-19 patients can also occur and lead to cardiac hypertrophy and injury through the activation of the innate immune response with release of proinflammatory cytokines leading to altered vascular permeability. **Huang et al** (doi.org/10.1016/ S0140-6736(20)30183-5) reported that 12% of patients with COVID-19 were diagnosed as having acute myocardial injury, manifested mainly by elevated levels of high-sensitive troponin I (Tnl). From other recent data, among 138 hospitalised patients, 16.7% had arrhythmias and 7,2% had acute myocardial injury.

In another case series, **Guo et al** (doi.org/10.1001%2Fjamacardio.2020.1017) reported that triage of patients with COVID-19 according to the presence of underlying cardiovascular disease or risks and the presence of elevated plasma cardiac biomarkers (N-terminal pro-brain natriuretic peptide and Troponin T levels) is needed to prioritize treatments and propose more aggressive treatment strategies in view of the increased risk for a fatal outcome and / or irreversible myocardial injury in these patients. While there are yet no studies about the COVID-19 long-term trajectory for surviving patients who have suffered from the most severe symptoms, it is anticipated that some patients will be predispsoed to suffer from long-term damage, including lung injury and scarring, heart damage, and neurological and mental health effects. Preliminary evidence, as well as historical research on other coronaviruses like severe acute respiratory syndrome (SARS CoV-1) and Middle East respiratory syndrome (MERS), suggests that for some patients, a full recovery might still be years off. Covid-19 survivors may experience long-lasting cardiac damage and cardiovascular problems, which could increase their risk for heart attack and stroke.

While there are many therapies being considered for use in treatment of COVID-19, there are as yet no approved safe and efficient medications to treat the disease, nor to prevent or cure the life-threatening complications of COVID 19 infections. No vaccine against SARS CoV has ever been developed. SARS Cov2 is an RNA virus and it is known that RNA virsues are more prone to changes and mutations compared to DNA viruses. Apart from the technological and manufacturing challenges, a successful vaccine strategy against COVID-19 is highly dependent on COVID-19 mutations and on how long the immunity will last against the virus. To date, treatment typically consists only of the available clinical mainstays of symptomatic management, oxygen therapy, with mechanical ventilation for patients with respiratory failure. Thus, to improve patient prognosis, there is an urgent need for novel therapies to address the different stages of the SARS-CoV-2 infectious cycle (**Siddiqi et al.** doi: 10.1016/j.healun.2020.03.012) and its life threatening consequences. Currently there is no evidence-based treatment and the use of experimental treatments may have cardiotoxic effects (such as hydroxychloroquine and azithromycine) or potent immune-suppressive effects (e.g. tocilizumab, corticosteroids, which may compromise the host immune response and viral clearance and which complicate the course of COVID-19 patients. There is a need for new effective and safe treatments addressing virus clearance and its wide range of symptoms for (1) patients with a confirmed diagnosis of SARS-CoV2 (2) for SARS-CoV2 infected people with preexisting heart problems to prevent worsening during SARS-CoV2 infection, (3) for at risk patients (e.g. elderly patients, patients with cardiac disease or patients, diabetic patients, patients with an underlying condition with a vulnerable heart...) who have an increased risk of morbidity and mortality, (4) in patients with perturbed coagulation biomarkers who are at greater risk to develop thrombotic events and, ultimately (5) for patients with damaged hearts from COVID-19 among those who survive from SARS-CoV2 infection.

### BRIEF DESCRIPTION OF THE DRAWINGS

**Figure 1a** shows the NHE-1 functioning under normal conditions.
**Figure 1b** shows the NHE-1 functioning under pathological conditions.
**Figure 2** shows a schematic for COVID-19 disease progression which includes two phases: 1) viral response phase and 2) host inflammatory response phase. There are also three stages roughly identified with the disease, with the most severe cases being in Stage III where patients suffer from a severe cytokine storm.
**Figure 3** shows Manifestations of COVID 19 cardiovascular complications and potential beneficial roles of NHE-1 inhibition.
**Figure 4** shows the chemical structures of amiloride and its analogue HMA compared to Rimeporide. The same atom numbering was used for the aromatice ring atoms for Amiloride, HMA and Rimeporide.
**Figure 5** shows the comparison of the main three structural features (moieties) for Amiloride, HMA, and Rimeporide.
**Figure 6** shows anti-inflammatory activity of rimeporide in skeletal muscles in male mdx mice in the forelimb and in the hindlimb.
**Figure 7** (a-e) shows the effects of rimeporide as well as other NHE-1 inhibitors on intracellular pH, intracellular Sodium and intracellular Calcium in accordance with Example 2.
**Figure 8** shows the dose dependency of rate constants to inhibit platelet swelling of 3 NHE-1 inhibitors (Eniporide, Cariporide and Rimeporide), in accordance with Example 3. (Platelet swelling in vitro).

### SUMMARY OF THE INVENTION

In one embodiment, the invention provides NHE-1 inhibitors, or pharmaceutically acceptable salts thereof, for use in the treatment of viral infections and their acute and chronic life-threatening complications in a subject in need thereof. In one aspect of this embodiment, the viral infection is a coronavirus infection. In one aspect of this embodiment, the viral infection is a SARS-CoV1, MERS-CoV, or SARS-CoV2 infection. In one aspect of this embodiment, the viral infection is a SARS-CoV2 infection.

One embodiment is a method of treating a coronavirus infected subject in need thereof, comprising administering an effective amount of an NHE-1 inhibitor, or a pharmaceutically acceptable salt thereof, to the subject. In one aspect of this embodiment, the subject has a confirmed diagnosis of COVID-19 pneumonia. In another aspect, the subject has COVID-19 myocardial injury, or the subject has COVID-19 and underlying cardiac diseases, wherein the term "cardiac disease" includes hypertension, coronary artery disease and diabetes. According to a further embodiment, the subject is suffering from cardiac complications with elevated cardiac markers of myocardial injury, e.g. elevated serum/ plasma levels of Troponin I /T, increased levels of NT-pro BNP, increased CRP, LDH or D-Dimer. In another aspect, the subject is suffering from a hyperinflammatory host immune response due to a SARS-CoV2 infection, from endothelial cells dysfunction, thrombosis, ALI and/or ARDS. In another embodiment, the subject has a confirmed diagnosis of COVID-19 and is at risk to develop severe form of COVID-19 because of age, underlying cardiac disease, hypertension, diabetes, coronary heart disease etc.

Another embodiment is an NHE-1 inhibitor, or a pharmaceutically acceptable salt thereof, for use in treating a coronavirus infected subject.

Another embodiment is the use of an NHE-1 inhibitor, or a pharmaceutically acceptable salt thereof, in the preparation of a medicament for the treatment of a coronavirus infected subject.

Because NHE-1 inhibitors have a general mode of action that restores the metabolism of the cells that are perturbed during COVID infections, NHE-1 inhibitors provide a unique approach to control the viral infection and prevent its wide range of symptoms in COVID 19 patients and in particular in those who are at risk of a severe form (e.g. older patients, patients with diabetes, with a vulnerable heart, with underlying cardiac disease).

Another emobodiment of the present invention is a method of treating a coronavirus infected subject in need thereof comprising administering a safe and effective amount of a NHE-1 inhibitor, or a pharmaceutically acceptable salts thereof, wherein the administration reduces the viral load in the subject. In one aspect of this embodiment, the NHE-1 inhibitor is administered in subjects with a confirmed diagnosis of COVID 19 to prevent developing a severe cytokine storm. In another aspect of this embodiment, the NHE-1 inhibitor is administered to prevent myocardial injury, arrythmia, myocarditis or heart failure, and in another embodiment, NHE-1 is administered to prevent thrombosis. In a further aspect of this embodiment, the subject has a mild to moderate SARS-CoV-2 infection and the NHE1 inhibitor is administered to prevent the development of heart failure, excessive host immune response and thrombosis. In an additional aspect of this embodiment, the subject has a confirmed diagnosis of SARS-CoV-2 but is asymptomatic at the start of the administration regimen but is predisposed to increased severity and mortality associated with the virus because of preexisting and underlying cardiac disease and its risk factors (diabetes and hypertension).

### DETAILED DESCRIPTION

Given the need for therapies to address the life-threatening complications of COVID-19, small molecule compounds, such as NHE-1 inhibitors, may be a valuable therapeutic intervention in providing relief to COVID-19 patients. The compounds of the invention are known to restore basic cell metabolism involving ion homeostasis and inflammation. Such compounds have shown antiviral properties through pH regulation and protein E interactions. The compounds have also shown benefit in improving inflammation, in preserving heart function, in preventing thrombosis, in protecting from fibrosis, without compromising viral clearance.

At the initial antiviral response phase (**Figure 2**), when the virus primarily infects ACE2-expressing specialized epithelial cells, direct anti-viral therapy may prove to be of benefit in minimizing contagion and preventing progression to severe disease (**Hoffmann, et al.** doi.org/10.1016/j.cell.2020.02.052; Sungnak, et al, Qbio preprint; arXiv:2003.06122; Zou, **et al**. doi.org/10.1007/s11684-020-0754-0; **Zhao, et al**.doi.org/10.1101/2020.01.26.919985,_**Qi**, **et al.** doi.org/10.1016/j.bbrc.2020.03.044; Taccone, et al. doi.org/10.1016/S2213-2600(20)30172-7). Indeed, recent papers have suggested a correlation between SARS-CoV-2 viral load, symptom severity and viral shedding (Liu, **et al**. doi.org/10.1016/S1473-3099(20)30232-2). Antiviral drugs administered at symptom onset to blunt coronavirus replication are in the testing phase (**Grein, et al.** doi: 10.1056/NEJMoa2007016. Taccone, et al).

Treatments addressing the severe complications of SARS-CoV-2 are proposed either in a staged approach to prevent excessive host immune response, cardiovascular, pulmonary complications and organ failure concomitantly with antivirals in patients with progressive disease (Fig 1, stage II) (**Stebbing, et al.** doi.org/10.1016/ S1473-3099(20)30132-8; **Richardson, et al.** doi.org/10.1016/S0140-6736(20)30304-4). Here, localized inflammation, systemic inflammatory markers, pulmonary and cardiovascular complications are more evident, necessitating more supportive care (e.g. hospitalization, oxygen supplementation) (**Siddiqi, et al**. doi: 10.1016/j.healun.2020.03.012). In this setting, anti-inflammatory therapies which are not compromising the patient's own ability to fight and clear virus load may be beneficial in preventing severe disease progression into a severe stage setting off a casacade of immune signals that can lead to multiorgans failure.

Cardiovascular complications are known and have already been described with MERS-CoV and SARS-CoV. Myocardial injury (MI) in SARS-CoV2 patients is not a common sequelae and optimal management for myocardial injury associated with COVID-19 patients has not been determined. It is based on supportive care using off label treatments. Chloroquine and azythromycine have to be used with great care in these patients as they are known to be cardiotoxic and/or to increase QTc intervals. In patients with COVID-19 infections, (**Guo et al**: doi.org/10.1001/jamacardio.2020.1017), NT-proBNP levels increased significantly during the course of hospitalization in those who ultimately died, but no such dynamic changes of NT-proBNP levels were evident in survivors. NT-proBNP elevation and malignant arrhythmias were significantly more common in patients with elevated TnT level, and NT-proBNP was significantly correlated with TnT levels. COVID-19 patients with myocardial injury are more likely to experience long term impairment in cardiac function.

Some current investigational immunomodulatory drugs are theorized to treat symptoms of cytokine storm associated with the host inflammatory phase of the illness (**Figure 2****,** Stage III). However, some medications currently being evaluated are too specific in their targeting to calm the cytokine storm (e.g., anti-IL6), too indiscrimate to be useful in calming the cytokine storm without causing too many adverse events (e.g., Jak1/2 inhibitors), are too weakly acting and/or non-specific in their targeting (e.g., hydroxychloroquine), and/or have serious side effects (**Richardson et al.** doi: 10.1001/jama.2020.6775; **Chen et al.** doi.org/10.1101/2020.03.22.20040758). However, none of the therapies currently being used in the clinic target the underlying driver to modulate the observed cytokine storm at its inception. Thus, even with multiple medications being evaluated in clinical trials, there is still a need for an effective therapeutic intervention to prevent or calm the cytokine storm without inhibiting the initiation of the host's immune defense mechanisms.

NHE-1 is a ubiquitous transporter and is the predominant isoform in the myocardium. This isoform of the antiport is primarily responsible for intracellular pH homeostasis and is involved in the regulation of cellular volume as well as in the regulation of inflammatory processes. It has been demonstrated in a number of inflammatory models that NHE-1 inhibition, using sabiporide (another NHE1 inhibitor discontinued for safety reasons) could significantly reduce NF-kB pathway activation, iNOS expression, chemokine production, leukocyte-endothelial cell interactions and attenuate neutrophil activation and infiltration (Dongmei et al. Plos One, 2013). Cariporide, another NHE-1 inhibitor, reduces the expression of ICAM1 and inhibits the leukocytes recruitment and adhesion as well as reduces the microvascular permeability in EE2 murine Endothelial cells (Qadri et al. Cardiovascular Diabetology 2014, 13:134).

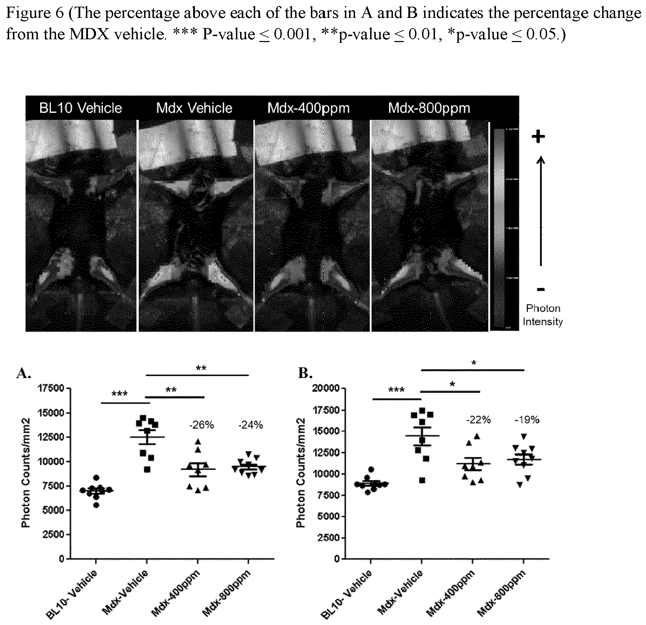

Rimeporide attenuated the postischemic impairment of myocardial function through reactive oxygen species-mediated ERK1/2/Akt/GSK-3β/eNOS pathways in isolated hearts from male Wistar rats. In vivo, potent anti-inflammatory activity of Rimeporide was shown in male mdx mice, a validated model of mice lacking dystrophin. Male wild-type and dystrophic mdx mice were treated for 5 weeks with vehicle, 400part per million (ppm) Rimeporide, or 800ppm. Rimeporide was mixed with their food starting at 3 weeks of age. At these 2 doses, plasma concentrations were ranging between 100 to 2500 ng/mL. Such concentrations can be achieved after oral dosing in human and have already been shown to be safe and well tolerated. After 5 weeks of treatment, there was a significant (and clinically relevant) reduction in inflammation in both the forelimb and hindlimb muscles as measured by optical imaging (**Baudy et al.** doi: 10.1007/s11307-010-0376-z) of all treated groups, with maximal reduction in Cathepsin B activity observed in the 400ppm Rimeporide treated group towards wild type levels (-26% for the forelimb and -22% for the hindlimb). See **Figure 6** which shows Cathepsin enzyme activity measurements via optical imaging. A) Forelimb photon count and B) Hindlimb photon counts as a direct measure of inflammation in 7-week-old Black10 mice, in Mdx mice receiving vehicle, and in treated Mdx mice treated 2 doses of rimeporide (400 and 800ppm). These findings confirm that Rimeporide significantly reduces skeletal muscle inflammation in vivo in mdx mice. These in vivo changes in skeletal muscle inflammation were confirmed by ex vivo histopathology (Hematoxylin & Eosin) studies. H&E analysis revealed a significant reduction in inflammation in the diaphragm with 400ppm Rimeporide treatment (44%) relative to vehicle in the mdx mice. Rimeporide also led to a significant prevention of inflammation in the diaphragm, a muscle of high relevance to Duchenne Muscualr Dystrophy (DMD) as the diaphragm exhibits a pattern of degeneration, fibrosis and severe functional deficit comparable to that of limb muscles in DMD patients where inflammation and fibrosis are present and contribute to the pathogenesis in addition to the lack of dystrophin the underlying cause of the disease.

An anti-inflammatory effect was confirmed in a clinical study with Rimeporide given orally for 4 weeks to young DMD boys (**Previtali et al**. doi.org/10.1016/j.phrs.2020.104999). A statistically significant decrease of MCP1, CCL15, TNFa, KLK6, Fas Ligand in the serum of patients receiving rimeporide for a 4-week treatment was observed in all dose groups. These chemokines are involved in monocytes adhesion to endothelial cells (**Park et al.** doi: 10.4049/jimmunol.1202284). Monocyte chemoattractant protein-1 (MCP-1/CCL2) is one of the key chemokines that regulate migration and infiltration of monocytes/macrophages. Both CCL2 and its receptor CCR2 have been demonstrated to be induced and involved in various inflammatory diseases and more recently in COVID-19 patients. Migration of monocytes from the blood stream across the vascular endothelium is required for routine immunological surveillance of tissues, as well as in response to inflammation (**Deshmane et al.** doi: 10.1089/jir.2008.0027)

**Table 1: Statistical analysis of plasma circulating biomarkers in patients with DMD after a 4 week treatment with Rimeporide (Abbreviation: FDR: False Discovery rate)**

| **Biomarker** | **Biomarker name** | **Source** | **Term** | **p-value** | **FDR** |
|---|---|---|---|---|---|
| CCL15 | C-C motif chemokine ligand 15 | OLink | -0.4489 | 0.0003 | 0.17 |
| KLK6 | Kallikrein 6 | OLink | -0.2612 | 0.0004 | 0.20 |
| FAS | Fas Ligand | OLink | -0.2823 | 0.0007 | 0.24 |
| TNFα | Tumor Necrosis Factor α | Luminex | -0.1726 | 0.0009 | 0.24 |
| MCP1 | Monocyte Chemoattractant Protein | OLink | -0.5675 | 0.0016 | 0.39 |

In Duchenne patients high levels of tumor necrosis factor alpha (TNF α), interferon (IFN)-y, and interleukin (IL)-12 are observed in the blood and muscle tissues. Myofibers are attacked by inflammatory cells at the endomysial, perimysial, and perivascular areas. Furthermore, a number of cytokines can exert direct effects on the muscle tissue via the activation of signaling pathways, such as the nuclear factor NF-kB pathway, which further enhances the inflammatory response through up-regulation of cytokine/chemokine production.

Similarly to DMD, a massive proinflammatory response after infection is the hallmark in severe cases of COVID-19 and contribute to disease severity and a worse prognosis (Chow, et al. Annu. Rev. Immunol. 2018. 36:667-94; Huang, et al. Lancet (2020), 395:497-506). In SARS-CoV-2 infected patients, retrospective analysis has demonstrated that initial plasma levels of IL-1β, IL-1RA, IL-7, IL-8, IL-10, IFN-y, monocyte chemoattractant peptide (MCP)-1, macrophage inflammatory protein (MIP)-1A, MIP-1B, granulocyte-colonystimulating factor (G-CSF), and tumor necrosis factor-alpha (TNF-α) are increased in patients with COVID-19 (**Tufan et al.** doi: 10.3906/sag-2004-168).

NHE-1inhibitors have the potential to prevent and protect against exuberant inflammatory response triggered by COVID-19 without impacting the host's immune response and the viral clearance. NHE-1 inhibitors are controlling the inflammatory response by preventing monocytes, macrophages and neutrophils accumulation and the excessive release of proinflammatory cytokines. NHE-1 transporters are expressed ubiquitiously and in particular in the heart, pulmonary endothelium and on lymphocytes CD4+ cells e.g. at the site of tissue injury caused by the virus. Rimeporide, a safe NHE-1 inhibitor, has the potential to specifically target the underlying mechanism of the cytokine storm observed in patients with COVID-19 and associated to a worse prognosis. Rimeporide, alone or in combination with other therapeutic intervention, could efficiently and safely modulate the inflammatory response without comprosing the host immune response against the SARS-CoV2.

Amiloride and its derivatives have demonstrated in vitro antiviral effects in other RNA virus infection. Holsey et al. (**Holsey et al.** doi.org/10.1002/jcp.1041420319) have shown that poliovirus infection (also a RNA virus) causes an increase of cytoplasmic pH which promotes virus production. EIPA inhibited both the pH rise and poliovirus production when added after virus absorption. Suzuki et al. (**Suzuki, 2001.** doi: 10.1152/ajprenal.2001.280.6.F1115.)) have shown that EIPA inhibits rhinovirus (HRV14) replication in human tracheal epithelial cells and decreases the number of acidic endosomes. Therefore, they concluded that its antiviral activity is due to the block of rhinovirus RNA entry from acidic endosomes to the cytoplasm. Suzuki et al. explained that as intracellular pH, endosomal pH is suggested to be regulated by ion transport across the endosomal V-ATPase and NHEs. They studied EIPA and FR-168888, inhibitors of NHEs, and bafilomycin A1, an inhibitor of Vacuolar H+ATPase (V-ATPase), reduced pHi and increased endosomal pH. Gazina et al (**Gazina et al.** doi:10.1016/j.antiviral.2005.05.003) confirmed the inhibition of rhinovirus release by these Amiloride derivatives and supported the idea that this stage of infection may become a target for antiviral agents. The mechanisms of the antiviral activity of amiloride derivatives is not elucidated, and according to Gazina et al. it is unlikely to be due to the inhibition of Na+ influx.

It is well known that viruses exploit and modify host-cell ion homeostasis in favor of viral infection. To that purpose, a wide range of highly pathogenic human viruses (such as SARS-CoV and MERS-CoV) encode viroporins. These are transmembrane proteins that stimulate crucial aspects of the viral life cycle through a variety of mechanisms. Noticeably, these proteins oligomerize in cell membranes to form ion conductive pores. Viroporins are involved in processes relevant for virus production. In general, these proteins do not affect viral genome replication, but stimulate other key aspects of the viral cycle such as entry, assembly, trafficking and release of viral particles. Ion channel (IC) activity may have a great impact on host-cell ionic milieus and physiology. Once inserted on cell membranes, viroporins tune ion permeability at different organelles to stimulate a variety of viral cycle stages. As a consequence, partial or total deletion of viroporins usually leads to significant decreases in viral yields. (**Nieto-Torres et al**: doi:10.3390/v7072786). IC activity ranges from almost essential, to highly or moderately necessary for viruses to yield properly.

CoVs' E protein has a viroporin-like activity. They are reported to oligomerize and form ion channels. While the S protein is involved in fusion with host membranes during entry into cells, and the M protein is important in envelope formation and bulding, E protein is not essential for *in vitro* and *in vivo* coronavirus replication. However, its absence results in an attenuated virus, as shown for SARS-CoV. (**Wilson et al.** doi:10.1016/j.virol.2006.05.028). Though the involvement of ion channels in CoVs pathogenesis is not fully understood, recently several studies have suggested that the absence of SARS-CoV E' protein results in an 'attenuated virus', thereby supporting that SARS-CoV E' protein is mainly responsible for pathogenesis and virulence. Interestingly, comparative sequence analysis reveals that SARS-CoV E' and 'SARS-CoV2 E' protein sequence share 94.74% identity amongst themselves (**Gupta et al:** doi: 10.1080/07391102.2020.1751300).

Besides modifying cellular processes to favor virus propagation/ pathogenicity, the loss of ion homeostasis triggered by viral ion conductivity activity may have deleterious consequences for the cell, from stress responses to apoptosis. That is why cells have evolved mechanisms to sense the ion imbalances caused by infections and elaborate immune responses to counteract viruses. Interestingly, the Ion Channel activity could trigger the activation of a macromolecular complex called the inflammasome, key in the stimulation of innate immunity. Inflammasomes control pathways essential in the resolution of viral infections. However, its disproportionate stimulation can lead to disease. In fact, disease worsening in several respiratory viruses infections is associated with inflammasome-driven immunopathology.

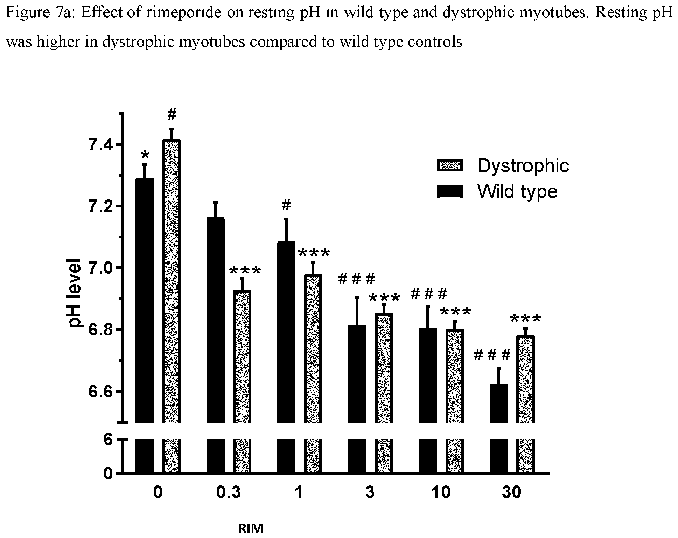

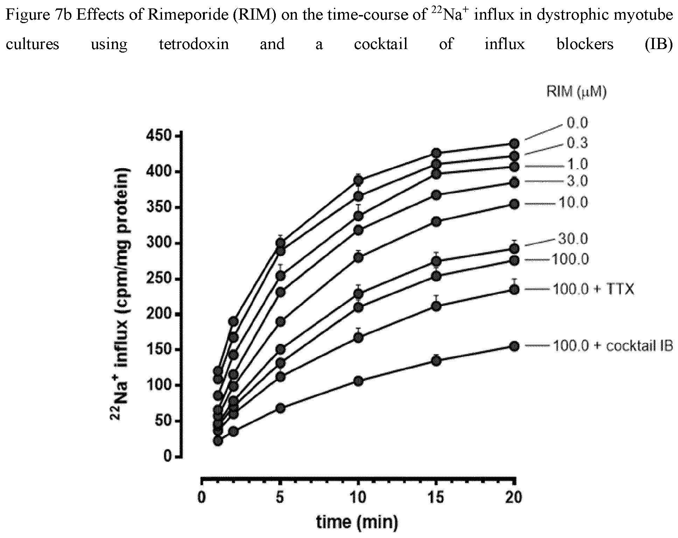

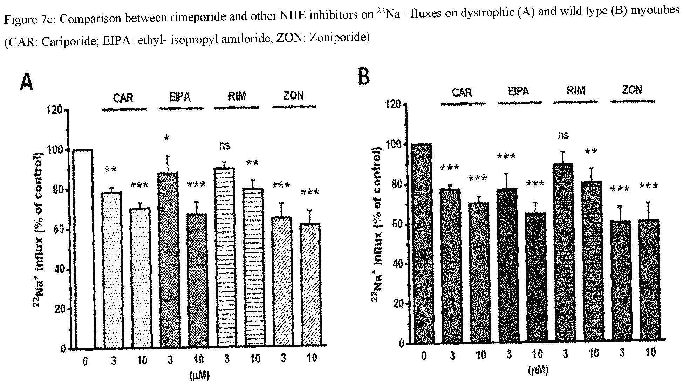

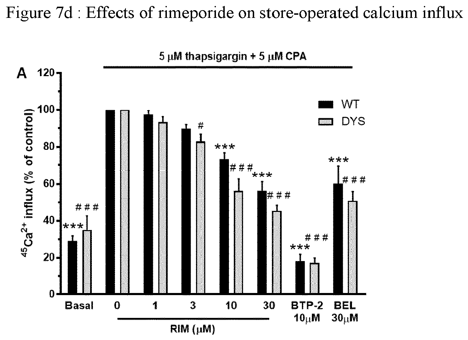

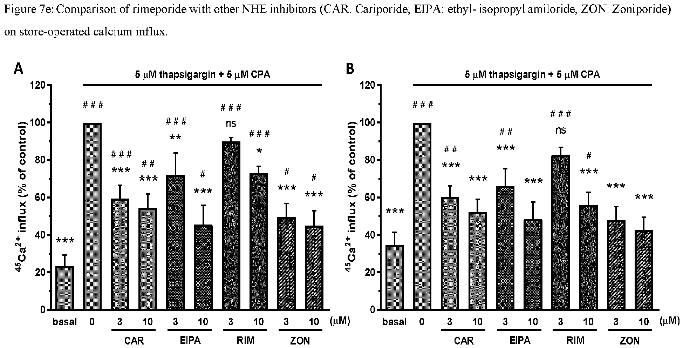

Taking into consideration the relevance of Ion channel activity in viral production, and its direct effect in pathology and disease, ion conductivity and its pathological stimulated pathways can represent targets for combined therapeutic interventions. The hexamethylene derivative of Amiloride (HMA) has been shown to block *in vitro* E protein ion channels and inhibit human coronavirus HCoV-229 E replication (Wilson et al. Virology 353 (2006) 294-306). SARS-CoV2 is an enveloped virus and E proteins present in them are reported to form ion channels which are an important trigger of immunopathology (Wilson et al; Virology 353 (2006) 294-306; **Gupta et al., 2020).** HMA inhibited in vitro ion channel activity of some synthetic coronavirus E proteins (including SARS-CoVE), and also viral replication (**Pervushin et al.** doi:10.1371/journal.ppat.1000511). Pervushin et al. 2009 demonstrated that HMA was found to bind inside the lumen of the ion channel, at both the C-terminal and the N-terminal openings and induced additional chemical shifts in the E protein transmembrane domain. This provides a strong rationale for ion channel activity inhibition. Inhibiting these ion channels and regulating pH and Calcium by Rimeporide (as shown in **Figure 7**) or with other NHE-1 inhibitors may thus help in controlling viral pathogenesis and propagation in humans.

SARS-CoV encodes three viroproteins: Open Reading Frame (ORF) 3a, protein E, and ORF 8a. Two of these viroporins, i.e., the more dominant protein E and also ORF 3a have ion channel activity which were reported to be required for optimal viral replication. The transmembrane domain of protein E forms pentameric alpha-helical bundles that are likely responsible for the observed ion channel activity. During the course of viral infections, these viroporins oligomerize and form pores that disrupt normal physiological homeostasis in host cell and thus contribute to the viral replication and pathogenicity. Shah et al (doi: 10.3389/fimmu.2020.01021) have shown that ion channel activity of protein E leads to the activation of the innate immune signaling receptor NLRP3 (NOD-, LRR-, and pyrin domain-containing 3) inflammasome through calcium release from intracellular stores. The activation of the inflammasome complex leads to the release of proinflammatory cytokines such as tumor necrosis factor alpha (TNF- alpha), IL-1 , and IL-6 ( Castano-Rodriguez et al. doi.org/10.1128/mBio.02325-17) and their accumulation promotes an exacerbated proinflammatory response, which leads to death. Due to this crucial role in triggering inflammatory response to infection, inhibition of protein E ion channel activity represents a novel drug target in the treatment of COVID-19 caused by SARS-CoV-2.

The present invention describes a structural analysis of chemical properties of various NHE-1 inhibitors' structures, their known electrostatic properties, as well as their affinity to bind inside E proteins' lumen using bio and cheminformatics approaches. Structural analysis and evaluation of chemical properties of NHE-1 inhibitors with regards to their inhibitory activities on E proteins, were performed using Chemistry and Biolnformatics approaches and tools. Based on a detailed analysis of the most eminent NHE-1 inhibitors presented in Example 1, Rimeporide is thought to have superior conformational and electrostatic properties to HMA and other NHE-1 inhibitors' in binding the inner lumen of E proteins of SARS-CoV, and thus may provide superior efficacy in inhibiting coronaviruses replication and pathogenicity via the inhibition of protein E's ion channel activity.

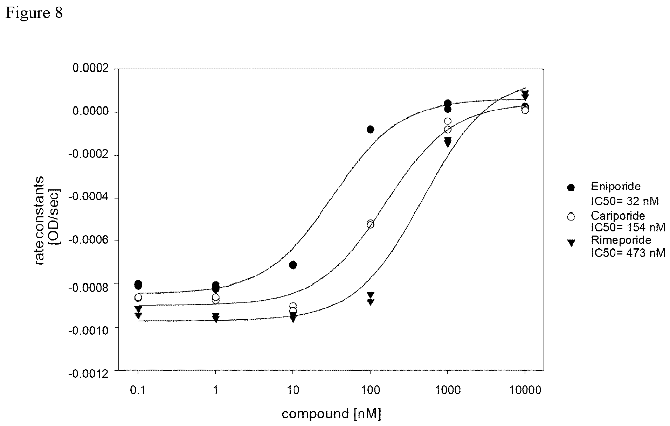

Rimeporide and other NHE-1 inhibitors have the potential to improve prognosis of patients with COVID-19 by decreasing platelet activation thereby preventing thrombosis which contributes to a worse outcome in patients. In fact, besides mediating hemostatic functions, platelets are increasingly recognized as important players of inflammation **(Mezger et al.** doi.org/10.3389/fimmu.2019.01731). Patients with COVID-19 often show clotting disorders, with end stage organ dysfunction and coagulopathy, thrombosis resulting in higher mortality. A dysregulated immune response, as seen in COVID-19, especially in the late stages of the disease, is known to play a decisive role in endothelial dysfunction and thrombosis and microvascular permeability is crucial in viral infections (**Mezger et al.2019**). NHE-1 plays a large role in platelet activation. Thrombus generation involves NHE-1 activation and an increase in intracellular Ca2+, which results from NHE-1-mediated Na+ overload and the reversal of the Na+/Ca2+ exchanger. Cariporide, a potent NHE-1 inhibitor, has inhibitory effects on the degranulation of human platelets, the formation of platelet-leukocyte-aggregates, and the activation of the GPIIb/IIIa receptor (PAC-1) (**Chang et al.** doi: 10.1016/j.expneurol.2014.12.023). As will be exemplified in more detail in Example 3, it is shown now by the present inventors that Rimeporide inhibits Calcium entry **(****Figure 7d** **and** **7e****)** and human Platelet swelling in an in vitro test using human blood and thus decreases the risk of thrombosis **(****Figure 8****)**. The platelet swelling inhibition capacity of Rimeporide is also shown in vivo in healthy subjects as exemplified in Example 4. Rimeporide therefore has the potential to improve COVID-19 patient prognosis by preventing thomboembolic events in patients with severe SARS CoV-2 infection alone or in combination with other anticoagulant therapies. In addition, as Rimeporide does not increase bleeding risks (as opposed to other anticoagulants such as aspirin, LMWH...), Rimeporide represents a safe therapeutic alternative to decrease thrombotic events in patients with contra indication to standard anticoagulants drugs.

NHE-1 plays an important role in Endothelial Cells (ECs) and inflammation as follows. NHE-1 is a ubiquitous transporter also present in the ECs, in the heart and in the lungs that regulates intracellular sodium, pH and indirectly calcium (Acta Physiol (Oxf). 2006 May-Jun; 187(1-2):149-57). NHE-1 is also involved in the regulation of inflammatory processes. It has been demonstrated in a number of inflammatory models that NHE-1 inhibition with Rimeporide could significantly reduce NF-kB pathway activation, iNOS expression, chemokine production, leukocyte-endothelial cell interactions and attenuate neutrophil activation and infiltration (**Dongmei et al.** doi:10.1371/journal.pone.0053932). NHE-1 blockade inhibits chemokine production and NF-kB activation in immune-stimulated endothelial cells (Nemeth et al. Am J Physiol Cell Physiol, 2002). Monocyte chemoattractant protein-1 (MCP-1/CCL2) is one of the key chemokines that regulate migration and infiltration of monocytes/macrophages. Both MCP1 and its receptor CCR2 have been demonstrated to be induced and involved in various diseases and to be increased in COVID-19 patients (**Tufan et al. doi:** 10.3906/sag-2004-168). Migration of monocytes from the blood stream across the vascular endothelium is required for routine immunological surveillance of tissues, as well as in response to inflammation (Deshmane et al. J of Interferon & Cytokine research, 2009). MCP1, CCL15, are two chemokines known to increase adhesion of monocytes to endothelial cells (**Park et al.** doi: 10.4049/jimmunol.1202284). CCL2, CCL15, KLK6 and TNFalpha were found to be decreased in the blood of DMD patients treated with Rimeporide after a 4-week treatment confirming Rimeporide's anti-inflammatory biological effect in DMD boys (Previtali **et al**. doi.org/10.1016/j.phrs.2020.104999). The involvement of Rimeporide in the regulation of ECs and inflammation without compromising the host immune response means that Rimeporide could be safely combined to other anti-viral and immunomodelating therapies for COVID-19 patients.

NHE-1 has an important role in cardiovascular pathophysiology. Increased activity and expression of NHE-1 plays a critical role in the pathogenesis of cardiac hypertrophy including heart failure, ischemia reperfusion injury. The role of NHE-1 in myocardial injury has been extensively studied. Ischemia causes intracellular acidification of cardiac myocytes, with reperfusion resulting in restoration of physiologic extracellular pH and creating a H+ gradient prompting efflux of H+, with concomitant Na+ influx through NH-1. The resultant rise in intracellular Na+ then prompts an increase in intracellular Ca2+ through the Na+/Ca2+ exchange system. Finally, elevated intracellular Ca2+ triggers deleterious downstream effects, including initiation of apoptotic pathways (Lazdunski, et al J Mol Cell Cardiol 1985;17(11):1029-1042). NHE-1 plays a critical role in cardiac hypertrophy and remodelling after injury. Indeed, cardiac-specific overexpression of NHE-1 is sufficient to induce cardiac hypertrophy and heart failure in mice (Nakamura et al:, Circ Res 2008;103(8):891-899). NHE-1 inhibition inhibits platelet activation and aggregation, lowering the risk of stroke (**Chang et al**.. doi: 10.1016/j.expneurol.2014.12.023). Rimeporide through NHE-1 inhibition is able to decrease the cardiovascular complications including heart failure, cardiac hypertrophy, necrosis and fibrosis in hereditary cardiomyopathic hamsters (**Chahine et al**, doi:10.1016/j.yjmcc.2005.01.003). Rimeporide through NHE-1 inhibition is able to prevent myocardial ischemia and reperfusion injury and attenuates post infarction heart failure in rats models of myocardial infarction (**Karmazyn et al**, doi: 10.1517/13543784.10.5.835, **Gazmuri 2019:** doi: 10.3390/molecules24091765). NHE-1 plays a role in myocardial injury during ischemia reperfusion. In rats model of heart failure following experimentally induced myocardial infarction (following permanent left coronary artery ligation), Rimeporide was shown to significantly and dose dependently reduce myocardial hypertrophy, preserve left ventricular function (as measured by cardiac output and left ventricular end diastolic pressure). Elevated gene expression of atrial natriuretic factor (ANP) NHE-1 were seen in untreated animals. ANP and its N-terminal precursor preproANP were decreased in the serum of treated animals in comparison to untreated animals. There was also a substantial improvement in survival in Rimeporide treated groups.

In a rat model of myocardial infarction (30 minutes coronary artery occlusion followed by 90 minutes reperfusion), Rimeporide given prophylactically (before occlusion) at doses ranging from 0,01 mg/kg to 1 mg/kg intravenously and 0,1 to 1 mg/kg per os was shown to reduce dose dependently infarct size. When given curatively (after the onset of ischemia and before reperfusion), Rimeporide reduced infarct size although higher dosages in comparison to prohylactic treatment are needed to achieve a similar reduction in infarct size.

In a model of anesthesized open-chest pigs of myocardial infarction (coronary artery occlusion for 60 minutes and subsequent 4 hours reperfusion), Rimeporide was able to decrease infarct size when given before coronary occlusion at 1 mg/kg iv and before reperfusion at 7 mg/kg iv. NHE-1 inhibition has been known for decades as potential treatment for myocardial ischemia.

In patients with COVID 19, there is a mismatch between oxygen supply and oxygen demand due to lung injury, patients may experience damage mimicking heart attack. Cardiac cells (including endothelial cells, cardiomyocytes and resident mast cells) respond to ischemia with release of mediators that influence myocardial performance. TNFalpha, IL6, IL1, IL8 and IL2 are part of a group of negative inotropic substances leading to a cardiodepressant effect. TNFalpha produces deleterious effect on left ventricular performance. Myocardial ischemia also results in intracellular acidosis. With restoration of coronary blood flow (reperfusion), the myocardium recovers from acidosis, at least in part, by activation of the NHE-1. NHE-1 activation leads to an increase in intracellular Na+ concentration, known to be responsible for cardiomyocytes hypertrophy. This ion abnormalities through NHE-1 increased activity causes an intracellular Ca2+ overload secondary to the activation of the Na+/Ca2+ exchange. Intracellular Ca2+ overload during early reperfusion is thought to be involved in the long-lasting depression of contractile function (stunned myocardium) and in the development of cell necrosis (ischemia/ reperfusion injury). SARS-CoV2 not only causes viral pneumonia but has major implications for the cardiovascular system. Patients with cardiovascular risk factors (including male sex, advanced age, diabetes, hypertension and obesity) and established cardiac diseases represent a vulnerable population when sufferring from COVID-19. Patients developing myocardial damage in the context of COVID-19 have an increased risk of morbidity and mortality. There is thus an urgent need to develop treatments for (1) patients with COVID-19 infections, (2) patients with COVID-19 infections with cardiac comorbidities, (3) patients with COVID-19 infections who develop myocardial injury as shown by elevated cardiac markers (Troponin or NT proBNP or other biomarkers predictive of myocardial injury), (4) patients with heart failure secondary to COVID-19 infection (5) patients with a vulnerable heart (preexisting underlying disease).

Acute lung Injury (ALI) is a major determining factor of the prognosis of patients with COVID-19 infection and precede . About 30% patients with COVID-19 infection in Intensive Care Unit (ICU) developed severe lung edema, dyspnea, hypoxemia, or even Acute Respiratory Distress Syndrone (ARDS). Lungs have been found to express NHE-1 (Orlowski et al J Biol Chem.Vol. 267, No. 13, Issue of May 5, pp. 9331-9339, 1992). Increased NHE-1 was observed in lung tissues from animals treated with Lipopolysaccharide (LPS). In the amiloride pretreatment (10 mg/kg Intravenous injection) group of rats, NHE-1 expression was significantly reduced and LPS induced lung injury was significantly inhibited. In line with the decrease NHE-1 expression, there was also a decreased p-ERK expression in amiloride treated rats. Inhibition of NHE-1 by Amiloride was shown to have a protective effect in a murine model of Lipopolysaccharide induced acute lung injury (**Zhang et al:** doi.org/10.1155/2018/3560234). In addition, NHE-1 is known to be activated in hypoxic rats in comparison to normoxic rats and NHE-1-/- mice are protected from hypoxia induced pulmonary artery hypertension (**Walker et al:** doi: 10.14814/phy2.12702). Rimeporide was shown to have an antifibrotic effect in the heart and the diaphragm when given in a preventive manner to dystrophic, mdx mice. NHE inhibition attenuates PASMC proliferation and migration in vivo in models of CH/SuHx pulmonary hypertension and protected from right ventricular failure Based on this, NHE-1 inhibitors such as Rimeporide could provide a lung protective effect in COVID-19 affected patients.

Diabetes (type 2 diabetes mellitus, T2DM) has been linked to increased susceptibility to and adverse outcomes associated with bacterial, mycotic, parasitic, and viral infections, attributed to a combination of dysregulated innate immunity and maladaptive inflammatory responses. Altered glucose homeostasis during a condition of severe pneumonia with SARS are reported as main factors of worse prognosis and deaths (Yang et al. Diabet Med. 2006 Jun;23(6):623-8). Both insulin resistance and T2DM are associated with endothelial dysfunction, and enhanced platelet aggregation and activation. T2DM and obesity are a frequent co-morbidities and a cause of worse prognosis / death in patients with COVID-19 infection (**Guan et al.** doi: 10.1056/NEJMoa2002032; **Sardu et al.** doi:10.20944/preprints202004.0204.v1). Endothelial cells NHE-1 are activated in patients with Type 2 diabetes (Qadri et al, 2014; Cardiovascular Diabetology 2014, 13:134). Excessive levels of methyglyoxal (MG: glycolysis metabolite) are encountered in diabetes and are responsible for the vascular complications including hypertension, enhanced microvascular permeability, and thrombosis. In endothelial cells, pathological concentrations of MG lead to activation of serum glucocorticoide inducible kinase 1 (SGK1) and to increased NHE-1. Cariporide attenuates the proinflammatory effects of excessive MG. NHE-inhibitors, such as Rimeporide, may be beneficial to prevent endothelial cell inflammation in COVID-19 patients with diabetes and hyperglycemia.

"COVID-19" is the name of the disease which is caused by a SARS-CoV2 infection. While care was taken to describe both the infection and disease with accurate terminology, "COVID-19" and "SARS-CoV2 infection" "COVID-19 pneumonia" are meant to be roughly equivalent terms.

As of the writing of this application, the determination and characteristics of the severity of COVID-19 patients/symptoms has not been definitively established. However, in the context of this invention, "mild to moderate" COVID-19 occurs when the subject presents as asymptomatic or with less severe clinical symptoms (e.g., low grade or no fever (<39.1°C), cough, mild to moderate discomfort) with no evidence of pneumonia, and generally does not require medical attention. When "moderate to severe" infection is referred to, generally patients present with more severe clinical symptoms (e.g., fever >39.1 °C, shortness of breath, persistant cough, pneumonia, etc.). As used herein "moderate to severe" infection typically requires medical intervention, including hospitalization. During the progression of disease, a subject can transition from "mild to moderate" to "moderate to severe" and back again in one course of bout of infection.

Treatment of subjects suffering from COVID-19 using the methods of this invention include administration of an effective amount of an NHE-1 inhibitor at any stage of the infection to prevent or reduce the symptoms associated therewith. Typically, subjects will be administered an effective amount of an NHE-1 inhibitor after definitive diagnosis and presentation with symptoms consistent with a SARS-CoV2 infection, and administration will reduce the severity of the infection and/or prevent progression of the infection to a more severe state. The clinical benefits upon such administration is described in more detail in the sections below.

### 1. Compounds and Definitions

In one embodiment of the invention the NHE-1 inhibitor is rimeporide hydrochloride or pharmaceutically acceptable salts thereof.

In another embodiment, the NHE-1 inhibitor is cariporide or a pharmaceutically acceptable salt thereof.

In another embodiment, the NHE-1 inhibitor is eniporide or a pharmaceutically acceptable salt thereof.

In another embodiment, the NHE-1 inhibitor is amiloride or a pharmaceutically acceptable salt thereof.

Unless otherwise stated, inhibitors mentioned herein are also meant to include compounds that differ only in the presence of one or more isotopically enriched atoms. For example, compounds having the present structures including the replacement of hydrogen by deuterium or tritium, or the replacement of a carbon by a ¹³C- or ¹⁴C-enriched carbon are within the scope of this invention. In some embodiments, the group comprises one or more deuterium atoms.

### 2. Uses, Formulation and Administration

The term "patient" or "subject", as used herein, means an animal, preferably a human. However, "subject" can include companion animals such as dogs and cats. In one embodiment, the subject is an adult human patient. In another embodiment, the subject is a pediatric patient. Pediatric patients include any human which is under the age of 18 at the start of treatment. Adult patients include any human which is age 18 and above at the start of treatment. In one embodiment, the subject is a member of a high-risk group, such as being over 65 years of age, immunocompromised humans of any age, humans with chronic lung conditions (such as, asthma, COPD, cystic fibrosis, etc.), humans with cardiac chronic conditions (such as heart failure, arrythmias, myocarditis, myocardial injury, myocardial fibrosis etc) and humans with other co-morbidities. In one aspect of this embodiment, the other co-morbidity is obesity, diabetes, and/or cardiovascular disease.

Compositions of the present invention are administered orally, parenterally, by inhalation spray, rectally, or nasally. Preferably, the compositions are administered orally. In one embodiment, the oral formulation of a compound of the invention is a tablet or capsule form. In another embodiment, the oral formulation is a solution or suspension which may be given to a subject in need thereof via mouth or nasogastric tube. Any oral formulations of the invention may be administered with or without food. In some embodiments, pharmaceutically acceptable compositions of this invention are administered without food. In other embodiments, pharmaceutically acceptable compositions of this invention are administered with food. In another embodiment, the NHE-1 inhibitor is inhaled using a drug powder inhaler.

In one embodiment, the intravenous formulation of a compound of the invention is an intravenous solution or a freeze-dried product developed for parenteral administration. In ventilated patients for whom it is not possible to administer the drug orally, a parenteral formulation of a compound of the invention could be administered intravenously as a slow release infusion or peritoneal route or via intramuscular injections.

Pharmaceutically acceptable compositions of this invention are orally administered in any orally acceptable dosage form. Exemplary oral dosage forms are capsules, tablets, aqueous suspensions or solutions. In the case of tablets for oral use, carriers commonly used include lactose and corn starch. Lubricating agents, such as magnesium stearate, are also typically added. For oral administration in a capsule form, useful diluents include lactose and dried cornstarch. When aqueous suspensions are required for oral use, the active ingredient is combined with emulsifying and suspending agents. If desired, certain sweetening, flavoring or coloring agents are optionally also added. Pharmaceutically acceptable compositions of this invention relates to pharmaceutical compositions for the parenteral administration of the compound in the form of sterile aqueous solutions provided a good stability or a lyophilized pharmaceutical solid composition to be reconstituted to provide a solution for intravenous, intraperitoneal and intramuscular administration.

In one embodiment, the formulation of a compound of the invention is provided as slow release formulation that allows to decrease the number of dosings per day. The amount of compounds of the present invention that are optionally combined with the carrier materials to produce a composition in a single dosage form will vary depending upon the host treated, the particular mode of administration (oral or parenteral). Preferably, provided compositions should be formulated so that a dosage of between 0.01 - 100 mg/kg body weight/day of the compound can be administered to a patient receiving these compositions.

In one embodiment, the total amount of NHE-1 inhibitor administered orally to the subject in need thereof is between about 50 mg to about 900 mg per day either as a single dose or as multiple doses.

In some of the above embodiments, the NHE-1 inhibitor is administered for a period of about 7 days to about 28 days.

In some of the above embodiments, the NHE-1 inhibitor is administered for a chronic period of more than 3 months due to a prolongation of the symptoms and the risks to develop severe and irreversible damages.

In one embodiment of the invention, the subject has a confirmed diagnosis of a COVID-19 infection. In one embodiment of the invention, the subject is suffering from an extreme proinflammatory response due to the COVID-19, which may present in any major organ of the body. In one embodiment of this invention, the subject is suffereing from acute respiratory distress syndrome (ARDS) due to COVID-19 and has elevated D Dimers or any other fibrinogen degradation products. In one embodiment of this invention, the subject is suffering from myocardial injury. In one embodiment of this invention, the subject is suffering from underlying cardiovascular disease and is predisposed to develop a severe form of COVID-19 infection. In one embodiment of this invention, the subject is suffering from one or more symptom chest pain, palpitations, syncope, hypertension, brady or tachy arrythmias, increased cardiac Troponin T/I, N-terminal B-type natriuretic peptide (NT pro BNP).

In one embodiment, the subject is suffering from a hyperinflammatory host immune response to a SARS-CoV2 infection. In one aspect of this embodiment, the hyperinflammatory host immune response is associated with one or more clinical indications selected from 1) reduced levels of lymphocytes, especially natural killer (NK) cells in peripheral blood; 2) high levels of inflammatory parameters (eg, C reactive protein [CRP], ferritin, d-dimer), and pro-inflammatory cytokines (eg, IL-6, TNF-alpha, IL-8, and/or IL-1beta; 3) a deteriorating immune system demonstrated by lymphocytopenia and/or atrophy of the spleen and lymph nodes, along with reduced lymphocytes in lymphoid organs; 4) dysfunction of the lung physiology represented by lung lesions infiltrated with monocytes, macrophages, platelets and/or neutrophils, but minimal lymphocytes infiltration resulting in decreased oxygenation of the blood; 5) acute respiratory distress syndrome (ARDS); 6) vasculitis; 7) encephalitis, Guillain-Barre syndrome, and other neurologic disorders; 8) kidney dysfunction and kidney failure; 9) hypercoagulability such as arterial thromboses; and 10) or any combination of above resulting in end-organ damage and death.

In one embodiment, the subject with COVID-19 is a pediatric patient suffering from vasculitis, including Kawasaki disease (i.e., Kawasaki syndrome) and Kawasaki-like disease.

In one embodiment of the invention, the subject is being treated inpatient in a hospital setting. In another embodiment, the subject is being treated in an outpatient setting. In one aspect of the preceding embodiments, the subject may continue being administered with the NHE-1 inhibitor after being transitioned from being treated from an inpatient hospital setting to an outpatient setting.

In one embodiment, the administration of the NHE-1 inhibitor results in one or more clinical benefit. In one aspect of this embodiment, the one or more clinical benefit is selected from the group comprising: reduction of duration of a hospital stay, reduction of the duration of time in the Intensive Care Unit (ICU), reduction in the likelihood of the subject being admitted to an ICU, reduction in the rate of mortality, reduction in the likelihood of reduction in the likelihood of heart failure, reduction in the likelihood of myocardial injury, reduction in the likelihood of acute lung injury, reduction of the time to recovery, reduction in the cytokine production, reduction of the severity of acute respiratory distress syndrome (ARDS), reduction in the likelihood of developing ARDS, reduction of the likelihood to have thrombotic events, and reduction of the excessive inflammatory response in the subject, reduction of the long term complications of COVID-19 infections including myocardial injury, lung injury and in particular pulmonary hypertension and kidney injury.

In one embodiment, the one or more clinical benefits includes the reduction of the inflammatory response of the subject. In one aspect of this embodiment, the reduction of the inflammatory response in the subject results in the reduction of proinflammatory cytokine release driven by NF□B (NF-kappa-B), ERK1/2, and includes MCP1 (or CCL2), TNFalpha, CCL15, KLK6. In one aspect of this embodiment, the one or more clinical benefits includes the prevention or the reduction or the avoidance of a severe cytokine storm in the subject.

In a further embodiment, the one of more clinical benefits is reduction in the likelihood of being hospitalized, reduction in the likelihood of ICU admission, reduction in the likelihood being intubated (invasive mechanical ventilation), reduction in the length of hospital stay, reduction in the likelihood of irreversible comorbidities including chronic heart failure, lung injury, kidney injury, reduction in the likelihood of mortality, and/or a reduction in likelihood of relapse, including the likelihood of rehospitalization.

The invention also provides a method of treating a viral infection in a subject in need thereof comprising administering an effective amount of an NHE-1 inhibitor to the subject. An amount effective to treat or inhibit a viral infection is an amount that will cause a reduction in one or more of the manifestations of viral infection, such as viral lesions, viral load, rate of virus production, and mortality as compared to untreated control subjects.

In one embodiment, the administration of the NHE-1 inhibitor selectively reduces the hyperinflammatory host immune response state while not interfering with the subject's innate immune response to the viral infection. In one aspect of this embodiment, the hyperinflammatory host immune response state is reduced before the subject suffers a severe cytokine storm.

One embodiment of the invention is a method of treating a coronavirus infected subject in need thereof, comprising administering an effective amount of a NHE-1 inhibitor, or a pharmaceutically acceptable salt thereof, to the subject. In one aspect of this embodiment, the subject is infected with SARS-CoV2. In another aspect of this embodiment, the administration of the NHE-1 inhibitor results in the reduction of the viral load in the subject.

In one embodiment, the NHE-1 inhibitor is administered prior to the subject developing a cytokine storm. In another embodiment, the subject has a mild to moderate SARS-CoV2 infection. In a further embodiment, the subject is asymptomatic at the start of the administration regimen. In another embodiment, the subject has had known contact with a patient who has been diagnosed with a SARS-CoV2 infection. In an additional embodiment, the subject begins administration of the NHE-1 inhibitor prior to being formally diagnosed with COVID-19.

One embodiment is a method of treating a subject with COVID-19 comprising administration of an effective amount of an NHE-1 inhibitor to the subject. In one aspect of this embodiment, the subject has been previously vaccinated with a SARS-CoV-2 vaccine and develops vaccine-related exacerbation of infection, for example, an antibody-dependent enhancement or related antibody-mediated mechanisms of vaccine/antibody-related exacerbation.

The NHE-1 inhibitors can be administered before or following an onset of SARS-CoV2 infection, or after acute infection has been diagnosed in a subject. A therapeutically relevant effect relieves to some extent one or more symptoms of a disorder, or returns to normality, either partially or completely, one or more physiological or biochemical parameters associated with or causative of a disease or pathological condition. The methods of the invention can also be used to reduce the likelihood of developing a disorder or even prevent the initiation of disorders associated with COVID-19 in advance of the manifestation of mild to moderate disease, or to treat the arising and continuing symptoms of an acute infection.

Treatment of mild to moderate COVID-19 is typically done in an outpatient setting. Treatment of moderate to severe COVID-19 is typically done inpatient in a hospital setting. Additionally, treatment can continue in an outpatient setting after a subject has been discharged from the hospital.

The invention furthermore describes a medicament comprising at least one NHE-1 inhibitor or a pharmaceutically acceptable salt thereof.

A "medicament" in the meaning of the invention is any agent in the field of medicine, which comprises one or more compounds of the invention or preparations thereof (e.g. a pharmaceutical composition or pharmaceutical formulation) and can be used in prophylaxis, therapy, follow-up or aftercare of patients who suffer from clinical symptoms and/or known exposure to COVID-19.

### Combination Treatment

In various embodiments, the active ingredient may be administered alone or in combination with one or more additional therapeutic agents. A synergistic or augmented effect may be achieved by using more than one compound in the pharmaceutical composition. The active ingredients can be used either simultaneously or sequentially.

In one embodiment, the NHE-1 inhibitor is administered in combination with one or more additional therapeutic agents. In one aspect of this embodiment, the one or more additional therapeutic agents is selected from antiviral, anti-inflammatories, antibiotics, anti-coagulants, antiparasitic agent, antiplatelets and dual antiplatelet therapy, angiotensin converting enzyme (ACE) inhibitors, angiotensin II receptor blockers, beta-blockers, statins and other combination cholesterol lowering agents, specific cytokine inhibitors, complement inhibitors, anti-VEGF treatments, JAK inhibitors, BTK inhibitors, immunomodulators, sphingosine-1 phosphate receptors binders, N-methyl-d-aspartate (NDMA) receptor glutamate receptor antagonists, corticosteroids, Granulocyte-macrophage colony-stimulating factor (GM-CSF), anti-GM-CSF, interferons, angiotensin receptor-neprilysin inhibitors, calcium channel blockers, vasodilators, diuretics, muscle relaxants, and antiviral medications.

In one embodiment, the NHE-1 inhibitor is administered in combination with an antiviral agent. In one aspect of this embodiment, the antiviral agent is remdesivir. In another aspect of this embodiment, the antiviral agent is lopinavir-ritonavir, alone or in combination with ribavirin and interferon-beta.

In one embodiment, the NHE-1 inhibitor is administered in combination with a broad-spectrum antibiotic.

In one embodiment, the NHE-1 inhibitor is administered in combination with chloroquine or hydroxychloroquine. In one aspect of this embodiment, the NHE-1 inhibitor is further combined with azithromycin.

In one embodiment, the NHE-1 inhibitor is administered in combination with interferon-1-beta (Rebif^{®}).

In one embodiment, the NHE-1 inhibitor is administered in combination with one or more additional therapeutic agents selected from hydroxychloroquine, chloroquine, ivermectin, tranexamic acid, nafamostat, virazole, ribavirin, lopinavir/ritonavir, favipiravir, arbidol, leronlimab, interferon beta-1a, interferon beta-1b, azithromycin, nitrazoxamide, lovastatin, clazakizumab, adalimumab, etanercept, golimumab, infliximab, sarilumab, tocilizumab, anakinra, emapalumab, pirfenidone, belimumab, rituximab, ocrelizumab, anifrolumab, ravulizumab, eculizumab, bevacizumab, heparin, enoxaparin, apremilast, coumadin, baricitinib, ruxolitinib, acalabrutinib, dapafliflozin, ibrutinib, evobrutinib, methotrexate, leflunomide, azathioprine, sulfasalazine, mycophenolate mofetil, colchicine, fingolimod, ifenprodil, prednisone, cortisol, dexamethasone, methylprednisolone, melatonin, otilimab, ATR-002, APN-01, camostat mesylate, brilacidin, IFX-1, PAX-1-001, BXT-25, NP-120, intravenous immunoglobulin (IVIG), and solnatide.

In one embodiment, the NHE-1 inhibitor is administered in combination with one or more anti-inflammatory agent. In one aspect of this embodiment, the anti-inflammatory agent is selected from corticosteroids, steroids, COX-2 inhibitors, and non-steroidal anti-inflammatory drugs (NSAID). In one aspect of this embodiment, the anti-inflammatory agent is diclofenac, etodolac, fenoprofen, flurbirprofen, ibuprofen, indomethacin, meclofenamate, mefenamic acid, meloxicam, nabumetone, naproxen, oxaprozin, piroxicam, sulindac, tolmetin, celecoxib, prednisone, hydrocortisone, fludocortisone, bethamethasone, prednisolone, triamcinolone, methylprednisone, dexamethasone, fluticasone, and budesonide (alone or in combination with formoterol, salmeterol, or vilanterol).

In one embodiment, the NHE-1 inhibitor is administered in combination with one or more immune modulators, with one or more anticoagulants.

In one embodiment, the NHE-1 inhibitor is administered in combination with one or more antibiotics. In one aspect of this embodiment, the antibiotic is a broad-spectrum antibiotic. In another aspect of this embodiment, the antibiotic is a penicillin, anti-straphylococcal penicillin, cephalosporin, aminopenicillin (commonly administered with a betalactamase inhibitor), monobactam, quinoline, aminoglycoside, lincosamide, macrolide, tetracycline, glycopeptide, antimetabolite or nitroimidazole. In a further aspect of this embodiment, the antibiotic is selected from penicillin G, oxacillin, amoxicillin, cefazolin, cephalexin, cephotetan, cefoxitin, ceftriazone, augmentin, amoxicillin, ampicillin (plus sulbactam), piperacillin (plus tazobactam), ertapenem, ciprofloxacin, imipenem, meropenem, levofloxacin, moxifloxacin, amikacin, clindamycin, azithromycin, doxycycline, vancomycin, Bactrim, and metronidazole.

In one embodiment, the NHE-1 inhibitor is administered in combination with one or more anti-coagulants. In one aspect of this embodiment, the anti-coagulant is selected from apixaban, dabigatran, edoxaban, heparin, rivaroxaban, and warfarin.

In one embodiment, the NHE-1 inhibitor is administered in combination with one or more antiplatelet agents and/or dual antiplatelet therapy. In one aspect of this embodiment, the antiplatelet agent and/or dual antiplatelet therapy is selected from aspirin, clopidogrel, dipyridamole, prasugrel, and ticagrelor.

In one embodiment, the NHE-1 inhibitor is administered in combination with one or more ACE inhibitors. In one aspect of this embodiment, the ACE inhibitor is selected from benazepril, captopril, enalapril, fosinopril, lisinopril, moexipril, perindopril, quinapril, ramipril and trandoliapril.

In one embodiment, the NHE-1 inhibitor is administered in combination with one or more angiotensin II receptor blockers. In one aspect of this embodiment, the angiotensin II receptor blocker is selected from azilsartan, candesartan, eprosartan, irbesartan, losartan, Olmesartan, telmisartan, and valsartan.

In one embodiment, the NHE-1 inhibitor is administered in combination with one or more beta-blockers. In one aspect of this embodiment, the beta-blocker is selected from acebutolol, atenolol, betaxolol, bisoprolol/hydrochlorothiazide, bisoprolol, metoprolol, nadolol, propranolol, and sotalol.

In another embodiment, the NHE-1 inhibitor is administered in combination with one or more alpha and beta-blocker. In one aspect of this embodiment, the alpha and beta-blocker is carvedilol or labetalol hydrochloride.

In one embodiment, the NHE-1 inhibitor is administered in combination with one or more interferons.

In one embodiment, the NHE-1 inhibitor is administered in combination with one or more angiotensin receptor-neprilysin inhibitors. In one aspect of this embodiment, the angiotensin receptor-neprilysin inhibitor is is sacubitril/valsartan.

In one embodiment, the NHE-1 inhibitor is administered in combination with one or more calcium channel blockers. In one aspect of this embodiment, the calcium channel blocker is selected from amlodipine, diltiazem, felodipine, nifedipine, nimodipine, nisoldipine, and verapamil.

In one embodiment, the NHE-1 inhibitor is administered in combination with one or more vasodilators. In one aspect of this embodiment, the one or more vasodilator is selected from isosorbide dinitrate, isosorbide mononitrate, nitroglycerin, and minoxidil.

In one embodiment, the NHE-1 inhibitor is administered in combination with one or more diuretics. In one aspect of this embodiment, the one or more diuretics is selected from acetazolamide, amiloride, bumetanide, chlorothiazide, chlorthalidone, furosemide, hydrochlorothiazide, indapamide, metalozone, spironolactone, and torsemide.

In one embodiment, the NHE-1 inhibitor is administered in combination with one or more muscle relaxants. In one aspect of this embodiment, the muscle relaxant is an antispasmodic or antispastic. In another aspect of this embodiment, the one or more muscle relaxants is selected from casisoprodol, chlorzoxazone, cyclobenzaprine, metaxalone, methocarbamol, orphenadrine, tizanidine, baclofen, dantrolene, and diazepam.

In some embodiments, the combination of a NHE-1 inhibitor with one or more additional therapeutic agents reduces the effective amount (including, but not limited to, dosage volume, dosage concentration, and/or total drug dose administered) of the NHE-1 inhibitor and/or the one or more additional therapeutic agents administered to achieve the same result as compared to the effective amount administered when the NHE-1 inhibitor or the additional therapeutic agent is administered alone. In some embodiments, the combination of an NHE-1 inhibitor with the additional therapeutic agent reduces the total duration of treatment compared to administration of the additional therapeutic agent alone. In some embodiments, the combination of an NHE-1 inhibitor with the additional therapeutic agent reduces the side effects associated with administration of the additional therapeutic agent alone. In some embodiments, the combination of an effective amount of the NHE-1 inhibitor with the additional therapeutic agent is more efficacious compared to an effective amount of the NHE-1 inhibitor or the additional therapeutic agent alone. In one embodiment, the combination of an effective amount of the NHE-1 inhibitor with the one or more additional therapeutic agent results in one or more additional clinical benefits than administration of either agent alone.

As used herein, the terms "treatment," "treat," and "treating" refer to reversing, alleviating, delaying the onset of, or inhibiting the progress of a viral infection, or one or more symptoms thereof, as described herein. In some embodiments, treatment is administered after one or more symptoms have developed. In other embodiments, treatment is administered in the absence of symptoms. For example, treatment is administered to a susceptible individual prior to the onset of symptoms (e.g., in light of a known exposure to an infected person and/or in light of comorbidities which are predictors for severe disease, or other susceptibility factors).

### EXEMPLIFICATION

### Example 1: Comparison of structural features of NHE-1 inhibitors with Amiloride and N-hexamethylene Amiloride

It has been shown in the literature that the activity of SARS-CoV envelope protein ion channels (SARS CoV E protein) can be inhibited by N-hexamethylene Amiloride (HMA) but not by Amiloride **(Pervushin et al.** doi:10.1371/journal.ppat. 1000511). This striking variation inhibiting E protein ion channel activity is observed despite the similarities in the structures (only one substituent at C5 position is different between the two structures). The impact of chemical structures differences of various NHE-1 inhibitors on the inhibitory potential of SARS CoV E proteins was evaluated in order to predict their ability to control SARS CoV pathogenicity and replication by inhibition of the ion channel activity. Among others, chemical structures were drawn with ChemBioDraw Ultra program, structural evaluations and optimizations of molecular structures were performed with MM2 Molecular Mechanics and Molecular Dynamics methods as implemented within the Chem3D Pro software. Biochemical libraries such as PubChem, RCSB Protein Data Bank and ChemSpider were also used to compare the ability of various NHE-1 inhibitors to block the ion channel activity of SARS-CoV through interactions with protein E, and thus to further predict direct inhibitory activity of replication and pathogenicity.

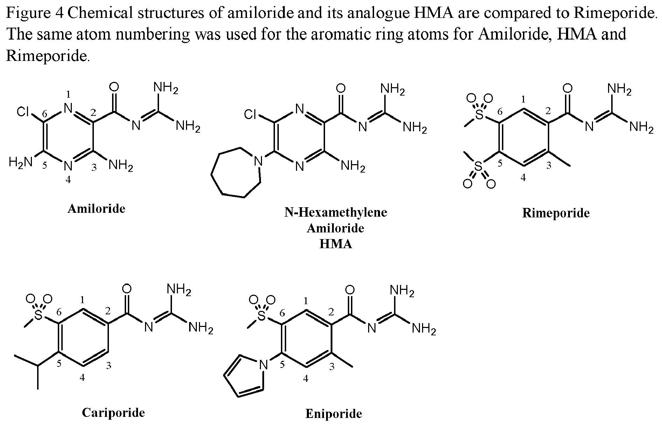

Pyrazine ring NHE-1 inhibitors (Amiloride and HMA) were compared to phenyl ring NHE-1 inhibitors (Rimeporide, Cariporide and Eniporide) (Figure 4).

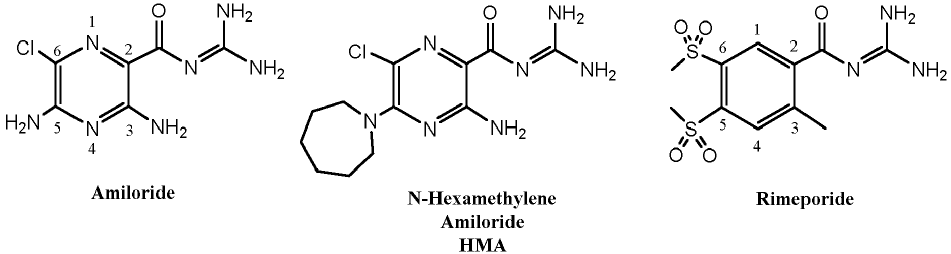

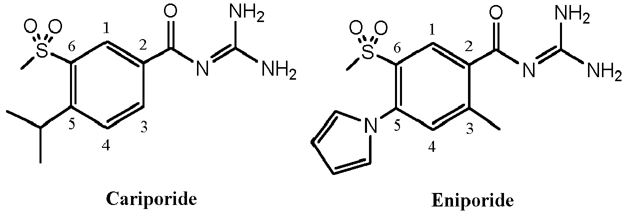

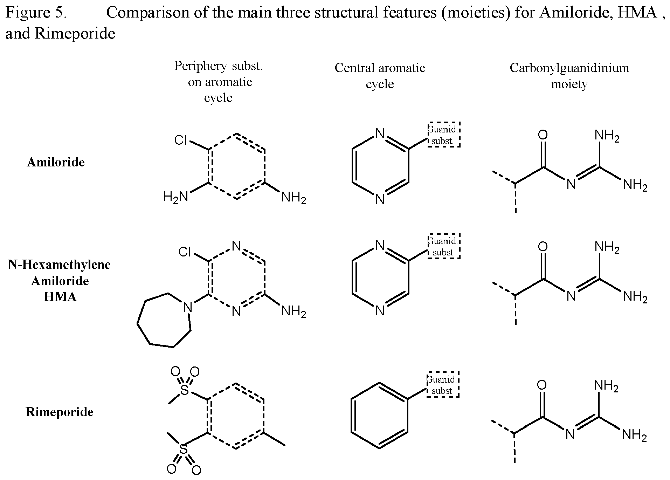

To facilitate the structural comparison, the three moieties are distinguished in chemical structures for Amiloride, HMA and Rimeporide compounds: ad A carbonyl-guanidinium moiety, ad B central aromatic ring, and ad C substituents on the periphery of the aromatic cycle. These moieties or structural features are shown in Figure 5 for each of the three compounds.

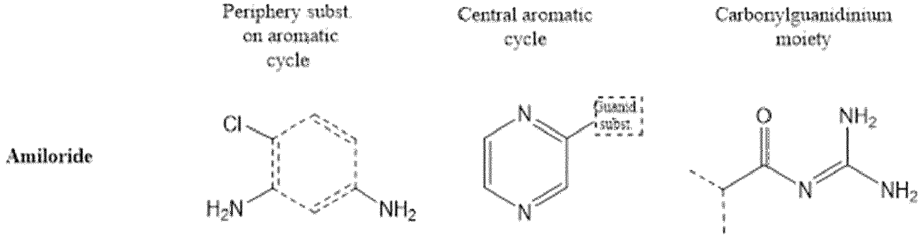

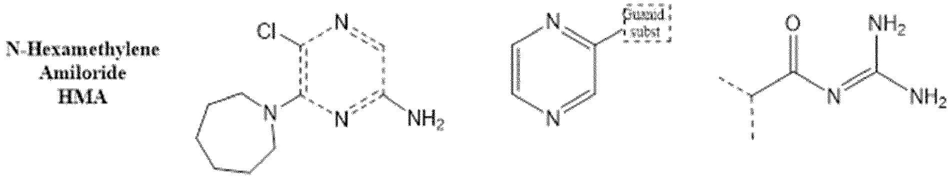

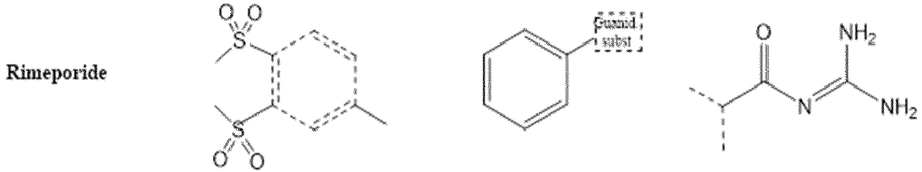

This work aimed to uncover the impact of each moiety and its various substituents of various NHE-1 inhibitors on their affinity to bind E protein lumen.

### Impact of the carbonyl-guanidinium moiety

Undoubtedly the guanidinium moiety plays an important role in the activity in respect to proteins. For example, in its protonated form the guanidinium binding is further stabilized through cation-π interactions. This moiety is also capable of directional H-bonding, and on other occasion can bind to an appropriate substrate through a salt bridge. In general, guanidinium can develop favorable interactions with numerous amino acid side chain and have the capacity to develop polyvalent interactions with proteins. For this moiety, the comparison between Amiloride, HMA and Rimeporide is straightforward as it is identical in all three structures.

### Impact of changes in the central aromatic cycle of NHE-1 inhibitors

For this moiety, both cycles: phenyl for Rimeporide and 1,4-pyrazine for Amiloride and HMA, are quite similar in their shape since in all cases is it is a planar, aromatic and stable six-membered ring. These properties warrant for all three structures in-plane special orientation of carbonyl-guanidinium and other peripheral substituents. Within this restriction, the variations can occur in meta-, ortho-, and para-substitution patterns. Amiloride and HMA own a pyrazine ring, where the two heterocyclic nitrogens modify the electron density on the remaining carbons of the cycle. Therefore, the carbons of pyrazine cycles are more electron-deficient than those of the phenyl ring of Rimeporide. The pyrazine ring is also more basic than phenyl in Rimeporide, nevertheless, the pyrazine is the least basic among diazine heterocycles and is clearly less basic than pyridine. It can be concluded that from structural and geometrical point of view that the phenyl and pyrazine cycles are quite similar while some differences exist in their electronic structures and basicity.

### Impact of different substituents on the periphery of the central aromatic ring of NHE-1 inhibitors

There is a significant variability of the substitution pattern on the periphery of the central aromatic cycle, other than preexisting carbonyl-guanidinium moiety. It has been shown that in the context of E-proteins these substituents influence the pathogenicity and the viral replication of SARS-CoV (**Pervushin et al.** doi:10.1371/journal.ppat.1000511). Close interactions were reported between the guanidinium residue located at R38 (arginine residue) with N-cyclohexamethylene cycle substituted at the C5 position of HMA deep inside the binding pocket located in the ion channel lumen in the C-terminal region of the E-protein. This contrast to the N-terminal binding site, where the guanidinium of HMA is inside the binding pocket, but the N-cyclohexamethylene is pointing away from the center of the channel. Our analysis has uncovered that the C-terminal binding site located in the vicinity of R38, is more pertinent then the N-terminal binding site to account for the observed selectivity, since only the binding at the C-terminal location allows to discriminate between the very different observed activities of Amiloride vs HMA. It appears that this large binding site can accommodate quite bulky C5 substituent such as N-cyclohexamethylene of HMA while comparatively small C5 amino substituent of Amiloride develop only reduced binding affinity. Indeed, HMA significantly reduced activity of E-proteins, while Amiloride was quasi-ineffective.

Methyl sulfone substituents (at the periphery of Rimeporide) have interesting physical and electronic properties such as dipole moments and dielectric constants which are indicative of pronounced polarity as a prerequisite for strong intermolecular interactions (T. Clark, at al., J. Mol. Mode.I 2008, 14:689). The electrostatic potentials on the molecular surface reveal interesting features including σ-holes on the sulfur therefore opening possibilities for variety of simultaneous intermolecular electrostatic interactions at the binding site inside the ion channel lumen.

Methyl sulfone substituents at the periphery of Rimeporide are much better suited for an efficient interaction with the guanidinium of R38 residue inside the ion channel lumen of E-proteins. Indeed, by comparison HMA possesses at the same C5 position a relatively inert 7-membered ring of N-cyclohexamethylene consisting of six methylenes and one nitrogen. The methyl sulfone substituent on Rimeporide is precisely located at the same C5-position (i.e. in para position in respect to the carbonyl guanidinium) of the six-membered aromatic ring. For example, for the series of sulfone analogues of COX-1 and COX-2 inhibitors, it has been shown in the literature that sulfones do bind efficiently to the arginine residues such as R38 (M. D. Pujol at al., Bioorg. Med. Chem, 2018, 26, 4113). Moreover, Rimeporide possesses a second methyl sulfone attached to the adjacent carbon ring C6. This invention argues for a distinct possibility that this second sulfone could improve even further the binding at the C-terminal binding site of E proteins and thus may strongly inhibit ion channel activity of E proteins essential in SARS CoV viral replication and virulence.

### Series of NHE-1 inhibitors as potential blockers SARS-CoV E-proteins activity.

In the context of substituents in the periphery of the aromatic ring, and inspired by dramatic variation of the activity of Amiloride vs HMA described by Pervushin (**Pervushin et al**. doi:10.1371/journal.ppat.1000511) other NHE-1 inhibitors, which are structurally related to Rimeporide, were also analysed. Namely, the comparison of Cariporide and Eniporide with Rimeporide (Figure 5), allows to better understand the role of different substituents - in particular at the C5-position to inhibit the ion channel activity. For the series of Cariporide, Eniporide and Rimeporide, the type of the C5 substituent changes dramatically while other structural features remain quasi-constant (phenyl ring is constant and C3-methyl is missing for Cariporide). This helps to finetune the understanding of the binding to SARS-CoV E-proteins lumen's with various NHE-1 inhibitors by finding the best arrangement of substituents in the periphery of the phenyl ring. For Cariporide, with the isopropyl as the three-carbon alkyl substituent at C5 for which the lower affinity can be anticipated towards the binding site where the guanidinium of arginine residue R38 plays a major role (Pervushin et al.). For the Eniporide,, where the C5 is involved in the N-phenyl substitution pattern, similarly to Rimeporide, Eniporide is an interesting candidate based on its steric, electrostatic and basicity properties.

In the context of this work, the goal was to explore the potential effects of various NHE-1 inhibitors on viral replication and pathogenicity via the inhibition of E proteins ion channel activity. This work has uncovered that the differences in C5 substituents have a major impact on the affinity of NHE-1 inhibitors to bind efficiently the lumen of E proteins and in particular with the C-terminal part of the protein. Among the 3 phenyl ring NHE-1 inhibitors studied (Cariporide, Eniporide and Rimeporide), Rimeporide appears as the best candidate for efficient binding inside the ion channel lumen of E protein, and for E protein ion channels activity inhibition.

### Example 2: Effects of rimeporide on intracellular pH, intracellular sodium and intracellular calcium

Fluorescence microscopy was used to monitor resting pH levels in primary dystrophic wild type myotubes through the use of the acetoxy methyl (AM) ester probe BCECF (Life technologies). To stimulate the activity of NHE, the cells were exposed to a transient 20 mM NH4CI pre-pulse. Effect of pretreatment with rimeporide on the resting pH levels and induced activity of NHE was also investigated. Myotubes were loaded with the pH probe BCECF-AM (5µM) for 20 min and allowed to de-esterify for another 20 min. Measurements were performed using non-radiometric fluorescence imaging microscopy by capturing images every 10 seconds with an excitation wavelength set at 440 nm and emission filter at 520 nm.

The effect of pretreatment with rimeporide on the resting Na+ level was also investigated. Fluorescence microscopy was used to monitor the response of the Na+ sensitive probe SBFI (Life technologies) in dystrophic and wild type primary myotubes. Using this experimental setting, an NH₄Cl pulse applied at the end of the pre-incubation step to activate NHE further had little impact on the net accumulation of ²²Na⁺. We hypothesized that efficacious efflux mechanisms prevented ²²Na⁺ to accumulate. Since Na⁺/K⁺-ATPase and Na⁺/Ca²⁺ exchanger (NCX) are major Na⁺ efflux, these transporters were blocked with respectively 1 mM ouabain (Sigma Aldrich) and 30 µM KB-R7943 (Tocris). The combination of NH₄Cl pulse and these efflux blockers resulted in the highest rate of ²²Na⁺ accumulation in dystrophic myotubes. This condition was chosen for the time-course experiments. Pretreatment with rimeporide (1-100 µM) resulted in a dose-dependent inhibition of ²²Na⁺ accumulation in both normal (not shown) and dystrophic myotubes. Rimeporide at 100 µM prevented total sodium accumulation by around 40% as measured after 10 to 20 min of influx. A comparison between rimeporide and other NHE inhibitors on ²²Na⁺ fluxes (CAR. Cariporide; EIPA: ethyl- isopropyl amiloride, ZON: Zoniporide) showed that Rimeporide was the weakest inhibitor as compared to other NHE-1 inhibitors (Zoniporide > Cariporide, EIPA > Rimeporide).

Effect of pretreatment with rimeporide on the resting Ca2+ level was also investigated. Incubating the myotube cultures with a calcium-free buffer containing thapsigargin and cyclopiazonic acid (CPA) caused Ca2+store depletion and activation of store-operated channels (SOC). Then, re-addition of Ca2+ in the extracellular medium caused a massive Ca2+ entry, known as store-operated calcium entry (SOCE). This resulted in a 3-3.5-fold increase in the net ⁴⁵Ca2+ accumulation compared to the basal condition in both wild type and dystrophic myotubes. The effect of Rimeporide was compared with other NHE-1 inhibitors (CAR: Cariporide; EIPA: ethyl-isopropyl amiloride; ZON: zoniporide). Rimeporide treatment induced an inhibition of SOCE in dystrophic and wild type myotubes. The direct SOC blocker BTP-2 at 10 µM induced an almost complete inhibition of Ca²⁺ flux induced by such a protocol. This novel effect of rimeporide has not been reported before and is shared with other NHE inhibitors. As observed for Na+ entry, Rimeporide was the weakest inhibitor of NHE-1 as compared to other NHE-1 inhibitors (Zoniporide > Cariporide, EIPA > Rimeporide)

Figure 7a shows the effects of Rimeporide on intracellular pH in accordance with this Example, as follows.: effect of rimeporide on resting pH in wild type (left bar) and dystrophic myotubes (right bar). Resting pH was higher in dystrophic myotubes compared to wild type controls (7.42±0.03 and 7.28±0.04 respectively). The regulation of intracellular pH in pathlogical conditions is thought to be a benefit in patients with COVID- 19 in several aspects : (1) **Bkaily,** doi.org/10.1139/cjpp-2017-0265) have underlined the significance of myocardial necrosis, due to pH abnormalities as well as calcium and sodium imbalances in the pathophysiology of heart failure and have demonstrated the beneficial effects of NHE-1 inhibition using Rimeporide in preventing the deleterious effects of Ca2+ and Na+ overload, (2) coronavirus entry into susceptible cells is a complex process that requires the concerted action of receptor-binding and pH-dependent proteolytic processing of the S protein to promote virus-cell fusion. So, the regulation of pH mediated by NHE-1 inhibition may be beneficial both on the viral infectivity and the fatal cardiovascular events triggered by the COVID-19 pneumonia.

Figure 7b shows the effects of rimeporide (RIM) on the time-course of ²²Na⁺ influx studied in wild type and dystrophic myotube cultures. The myotube cultures were treated with Rimeporide during pre-incubation (20 min) and during influx (1 to 20 min) at concentrations ranging from 0 (control) to 100 µM. Rimeporide dose-dependently inhibited the net accumulation of ²²Na⁺ into the myotube cultures. The contribution of other pathways to Na⁺ influx was examined using several pharmacological tools. The addition of tetrodotoxin (TTX), a blocker of voltage-gated sodium channels Nav.1.4 to 100 µM Rimeporide further decreased the influx by around 8%. Finally, a cocktail of influx blocker (IB) containing rimeporide and TTX was used to also block muscarinic acetylcholine receptors, nicotinic acetylcholine receptors, sodium-potassium-chloride co-transporters (NKCCs), transient receptor potential (TRP) cationic channels, and non-selective calcium channels showing sodium conductance, respectively. This further inhibited ²²Na⁺ influx by around 27%. Rimeporide alone inhibited Na⁺ influx by around 40% demonstrating that NHE is the major Na⁺ influx pathway in myotube cultures.

Figure 7c shows a comparison between rimeporide and other NHE inhibitors on ²²Na⁺ fluxes (CAR: Cariporide; EIPA: ethyl- isopropyl amiloride, ZON: Zoniporide).

The regulation of intracellular Na+ in patients with COVID-19 may be beneficial to several complications of COVID-19 infections by (1) preventing platelet activation and thereby preventing thrombotic events, (2) preventing the cardiac hypertrophy seen in patients with myocarditis (3) protecting from the increased lung permeability that results from the inflammatory injury to the alveolo-capillary membrane and leading in the end to respiratory insufficiency.

Figure 7d shows the effect of Rimeporide on calcium store operated channel entry (SOCE). Rimeporide dose-dependently prevented SOCE in both wild type and dystrophic myotubes (differentiation day +7 ± 1). Rimeporide mediated inhibition of SOCE tended to be more pronounced in dystrophic myotubes than in wild type ones, with statistical differences found as low as 3 µM, and strong inhibition reaching basal levels at 30 µM in dystrophic myotubes. In fact, in both cultures Rimeporide reduced SOCE with a similar efficacy as BEL (Bromo-Enol Lactone (BEL, an inhibitor of the calcium-independent phospholipase A2, iPLA2) that indirectly prevents SOCE via inhibition of iPLA2. SOCE was abolished by BTP-2, a compound that blocks Orai channels directly and is an inhibitor of store-operated channels.

Figure 7e shows a comparison between Rimeporide (RIM) and other NHE inhibitors ( CAR: Cariporide; EIPA: ethyl-isopropyl amiloride; ZON: zoniporide) on SOCE.

Regulation of calcium entry by NHE-1 inhibitors may have a role on several pathogenesis of COVID-19 infection: (1) NHE-1 plays a large role in platelet activation. Thrombus generation involves NHE-1 activation and an increase in intracellular Ca2+, which results from NHE-1-mediated Na+ overload and the reversal of the Na+/Ca2+ exchanger. The present inventors show that Rimeporide, through inhibition of calcium entry is able to prevent thrombotic complications in COVID-19 patients. Calcium entry (**Figure 7d****,** **7e**); (2) by regulating Calcium influx, Rimeporide and other NHE inhibitors have been shown to prevent from myocardial injury I several animal models (mdx mice, GRMD dogs, cardiomyopathic hamsters).

### Example 3: In vitro evaluation of the inhibition of human platelet swelling by the NHE inhibitors Rimeporide, Eniporide and Cariporide

The intracellular acidification, subsequent to hypoxemia, causes activation of the Na+/H+- exchange (NHE), which in turn contributes to the uptake of Na+ ions and (obligatory) water molecules. This induces the swelling of the platelets leading to an increased platelet volume and size. Larger platelets are hemodynamically more active and represent an increased risk for thrombosis. The platelet swelling assay served as a pharmacodynamic biomarker of drug activity. Platelets respond to an intracellular acid challenge by activating plasmalemmal NHE. The uptake of Na+ ions together with free water molecules causes a swelling of the platelets. To test the potential of Rimeporide, Cariporide and Eniporide of inhibiting human platelet swelling, the following experiments were performed.

500 µl of the platelet rich plasma containing 2.4 x 108 cells (appropriately diluted using platelet free plasma) were given into a plastic cuvette (1 cm path length), which was placed in a Hitachi U-2000 double beam spectrophotometer. Thereafter 1500 µl of the incubation buffer (± appropriately diluted compound) was added. 1 mM stock solutions of the compounds in DMSO were prepared. Thereafter an appropriate aliquot of the stock solution was diluted 100-fold in the incubation buffer. Further dilutions were made using the incubation buffer containing in addition 1 % DMSO. The final buffer component concentrations were (mM): Na-propionate 90, K-propionate 15, HEPES 15, glucose 7.5, KCI 3.7, MgCl2 0.75, CaCl2 0.75, 0.75 % DMSO, pH 6.6; the thrombocyte concentration was therefore 6 x 107 cells/ml. After the addition of the buffer the solution in the cuvette was mixed by moving a plastic cuvette mixer slowly 1 time up and down.

The change in absorbance at 680 nm was followed for 2 min 20 sec; the absorption values were collected every 10 sec. The platelet swelling induces a decrease in the absorbance. The decrease in optical density is thought to be induced by the diffusion of the undissociated form of the weak organic acid, propionic acid, into the cytoplasm of the thrombocytes, where it contributes to a decrease in the intracellular pH (pHi).

Figure 8 shows the concentration dependence of the rate constants derived from the linear regression analysis of a plot of the natural logarithm of the normalized OD data (obtained at 680 nm) against time using the platelet swelling assay.

Though with a different degree of potency, all 3 NHE inhibitors exhibited a similar behaviour in reducing the decrease in optical density at 680 nm which was observed in an optical swelling assay using human platelet rich plasma.

The decrease in optical density is thought to be induced by the diffusion of the undissociated form of the weak organic acid, propionic acid, into the cytoplasm of the thrombocytes, where it contributes to a decrease in the intracellular pH (pHi). The intracellular acidification causes activation of NHE, which in turn contributes to the uptake of Na+ ions and (obligatory) water molecules.

The measured optical density values (OD) were transformed into a set of linearised and normalised data which are representative of the change in absorbance by subtracting from all OD(t)values the ODt=4'-value and dividing the result by the OD-value of the starting time point

It is obvious that Eniporide is the most potent compound, with an IC50 (+ SEM) of 30 +/- 1 nM. Rimeporide exhibits a mean IC50 of 455 +/-36 nM and Cariporide inhibits the platelet swelling with an IC50-value of 166 +/-22 nM.

### Example 4: Ex vivo evaluation of the inhibition of human platelet swelling by the NHE inhibitors Rimeporide and Eniporide

### Eniporide ex vivo anti-swelling effect results

Six phase I studies were performed to provide information on safety and tolerability, pharmacokinetics, pharmacodynamics and metabolism of Eniporide and its main metabolite in healthy subjects. Clinical studies were designed as double-blind, placebo-controlled, randomised, sequential group, single rising, intravenous dose either as a bolus or as an infusion. In patients treated with eniporide, platelet swelling was reduced from a dose of 5 mg onwards. For 50 mg and above, total inhibition of platelet swelling was observed immediately post dosing. More than 80% inhibitory activity remained at 1.5 or 3 hours post dose for doses 100 - 200 mg; and 250 - 400 mg respectively which corresponds to the elimination half life of eniporide. After administration of 2 times 200 mg, as an infusion, 80% of the inhibitory activity was seen at 6 hours post first dose. No clinically relevant effect on platelet aggregation was noted up to 100 mg Eniporide and no clinically relevant effects on PT (prothrombin time) or APTT (activated partial thromboplastin time) were observed. Higher doses were not tested.

### Rimeporide ex vivo anti-swelling effects results

Platelet swelling was measured in 2 clinical studies (1 single oral ascending dose study and 1 multiple oral ascending dose study).

### Single oral ascending dose study with Rimeporide EMD 62 204-004

The study was designed as a double-blind, placebo-controlled, oral, single rising dose study on the safety, tolerability, pharmacokinetics and pharmacodynamics of Rimeporide in healthy male volunteers. Within each dose group (from 300 to 600 mg), six subjects were randomized to receive oral treatment with Rimeporide and three with placebo.

In addition to safety and pharmacokinetic measures, platelet swelling, a pharmacodynamic marker, was measured ex vivo in blood samples collected from the subjects pre-dose and at 1, 3, 6, 12, 24 and 48 hours post-dose. Rate constants of the platelet swelling reaction were calculated for each of the samples. The plasma concentrations of Rimeporide were plotted against study time on the same figures as the rate constants for the subjects receiving Rimeporide. As an indication of inhibition of platelet swelling the rate constants measured in the post-dose blood samples were compared with the corresponding rate constants measured in the pre-dose (baseline) blood samples for individual subjects. A post-dose decrease in rate constant was consistently observed for the subjects receiving Rimeporide, no such change in rate constant was observed for subjects receiving placebo. A decrease in the rate constant was generally present at 1 hour post-dose, the first sample time point. The exception was Subject 15, in this subject the rate constant at 1 hour post-dose was similar to the pre-dose value, a decrease in the rate constant was not observed until 3 hours post-dose. The plasma concentration of Rimeporide in this subject was 159 ng.mL-1 at 1 hour post-dose and 3690 ng.mL-1 at 3 hours post-dose, the plasma concentrations of Rimeporide in the other subjects at 1 hour post-dose were in the range 2320 to 10500 ng mL-1.

In the majority of subjects the decrease in rate constant was maintained at 3 hours postdose, plasma concentrations of Rimeporide at 3 hours post-dose were in the range 1680 to 5390 ng.mL-1. At 12 hours post-dose the rate constants were similar to Predose values, plasma concentrations of Rimeporide at 12 hours post-dose were in the range 160 to 965 ng mL-1. These results suggest that Rimeporide inhibits platelet swelling and that the lower Rimeporide plasma concentration threshold for inhibition of platelet swelling is between 965 to 1680 ng mL-1 which is within the range of plasma concentration that can be achieved safely with an oral treatment.

### Platelet aggregation and Prothrombin Time (PT) and activated Partial Thromboplastin Time (APTT)

Platelet aggregation following the addition of 10 µM ADP to diluted platelets (240-300x 103 platelets/µL) was measured ex vivo pre-dose and at 3, 24 and 48 hours post-dose.

There was no evidence of inhibition of platelet aggregation following the administration of Rimeporide or placebo at any of the time points tested. No effects of Rimeporide were observed on the PT and APTT results.

This means that Rimeporide is able to inhibit platelet swelling without compromising the coagulation parameters.

### Multiple ascending dose study (EMD 62 204-002):

Blood samples collected from the subjects on day 3 at 2.5, 4.5, 7.5, 10.5, 12.5, and 15.5 hours following the first dose administration, day 5 pre-dose and 2.5 h post-dose, and on day 10 at 2.5, 4.5 and 7.5 hours post-dose were used for the ex vivo platelet swelling assy. Rate constants of the platelet swelling reaction were calculated for each of the samples and the inhibition of platelet swelling was calculated from these values and the subjects calibration curve.
In this study different concentrations of Rimeporide were used in the preparation of the calibration curves (1, 10, 100, 500, 1000, 5000, 10000 and 100000 nM) and a clear sigmoidal relationship between rate constant and Rimeporide concentration was demonstrated at these concentrations. Inspection of individual subject platelet swelling inhibition profiles for day 3 indicate some evidence of inhibition albeit inconsistent in 67% of subjects receiving Rimeporide, the inhibition is more noticeable following the second dose of Rimeporide. Overall the results are difficult to interpret but there does appear to be evidence of inhibition of platelet swelling in the pm samples on day 3 which would approximately coincide with the maximum plasma concentration of Rimeporide following the pm dose administration.

Thrombosis has been shown to contribute to increased mortality in COVID-19 patients. It can lead to a pulmonary embolism (PE), which can be fatal, but also higher rates of strokes and heart attacks are observed in patients with thrombosis. This was confirmed in several retrospective studies and provides a rationale for using anticoagulant therapies to prevent from thrombosis.

Rimeporide is able to efficiently reduce the swelling of platelets, thereby decreasing platetets activation without compromising the coagulation parameters.

Dysregulated immune response, as seen in COVID-19, especially in the late stages of the disease, is known to play a decisive role in endothelial dysfunction and thrombosis and microvascular permeability is crucial in viral infections **(Mezger et al.2019).** The platelet swelling inhibition capacity of Rimeporide **(****Figure 8****),** also shown in vivo in healthy subjects is promising for patients with COVID-19 in particular in those who have a bleeding risk. Rimeporide therefore represents a safe therapeutic combination therapy and/or a safe alternative to anticoagulantsdecrease thrombotic events in patients COVID-19.

## Claims

1. A method of treating a coronavirus infected subject in need thereof, comprising administering an effective amount of an NHE-1 inhibitor, or a pharmaceutically acceptable salt thereof, to the subject.

2. The method of claim 1, wherein the coronavirus causes a SARS or MERS infection.

3. The method of claim 1 or 2, wherein the coronavirus causes a SARS-CoV1 or SARS-CoV2 or MERS-CoV infection.

4. The method of any one of claims 1-3, wherein the coronavirus is SARS-CoV2.

5. The method of any one of claims 1-4, wherein the NHE-1 inhibitor is selected from the group consisting of rimeporide, cariporide, eniporide, amiloride or a pharmaceutically acceptable salt thereof.

6. The method of claim 5, wherein the NHE-1 inhibitor is rimeporide.

7. The method of any one of claims 4-6, wherein the subject is suffering from a hyperinflammatory host immune response to a SARS-CoV2 infection.

8. The method of any one of the preceding claims wherein the subject has a moderate to severe SARS-CoV2 which requires medical intervention.

9. The method of any one of the preceding claims, wherein the subject has COVID-19 myocardial injury.

10. The method of any one of the preceding claims, wherein the subject has COVID-19 and a diagnosed or undiagnosed cardiac disease, including hypertension, coronary artery disease, heart failure and diabetes.

11. The method of any one of claims 7-10, wherein the hyperinflammatory host immune response is associated with one or more clinical indications selected from 1) increased laboratory markers suggestive of myocardial injury including elevated serum/ plasma levels of Troponin I /T, increased levels of NT-pro BNP, increased CRP, LDH or D-Dimer 2) high levels of inflammatory parameters (eg. C reactive protein [CRP], ferritin, d-dimer), and pro-inflammatory cytokines (eg, IL-6, TNFα, CCL2, CCL15, IL-8, and/or IL-1); 3) dysfunction of the lung physiology represented by diffuse pulmonary intravascular coagulopathy, lung lesions infiltrated with monocytes, macrophages, and/or neutrophils, but minimal lymphocytes infiltration resulting in decreased oxygenation of the blood; 4) acute respiratory distress syndrome (ARDS); 5) vasculitis, and 6) hypercoagulability such as arterial thromboses, or any combination of the above resulting in end-organ damage and death.

12. The method of any one of the preceding claims, wherein the subject is an adult patient or a pediatric patient.

13. The method of any one of claims 1-12 wherein the NHE-1 inhibitor is administered via oral route

14. The method of any one of claims 1-12 wherein the NHE-1 inhibitor is administered via parenteral or topical or inhalational routes.

15. The method of any one of the claims 1-14 wherein the NHE-1 inhibitor in administered acutely or chronically.
